# EUROPEAN PATENT SPECIFICATION

(11) **EP 0 744 027 B1**
(45) Date of publication and mention of the grant of the patent: **16.03.2005**
(21) Application number: 95908522.6
(22) Date of filing: 12.01.1995
(51) Int. Cl.: G01N 33/531, G01N 33/68

(54) **USE OF METHODS FOR IDENTIFYING HYDROPHILIC SIGNAL OLIGOPEPTIDES**
VERWENDUNG VON METHODEN ZUR IDENTIFIZIERUNG VON HYDROPHILEN SIGNAL-OLIGOPEPTIDEN
UTILISATION DES METHODES POUR IDENTIFIER DES OLIGOPEPTIDES SIGNAUX HYDROPHILES

(30) Priority: 14.01.1994 US 182248
(43) Date of publication of application: 27.11.1996
(60) Divisional application: 04030374.5
(73) Proprietor: Rath, Matthias, Dr. med., 7609 KL Almelo (NL)
(72) Inventor: Rath, Matthias, Dr. med., 7609 KL Almelo (NL)
(74) Representative: Federhen, Ludwig, Dr.
(86) International application number: PCT/US1995/000575
(87) International publication number: WO 1995/019568

(56) References cited:
- WO-A-89/05823
- WO-A-89/10405
- US-A- 4 554 101
- JOURNAL OF APPLIED NUTRITION, Volume 44, Numbers 3 & 4, issued 1992, RATH, "Cationic-Anionic and Anionic-Cationic Oligopeptides in Apoprotein-a and other Proteins as Modulators of Protein Action and of Biological Communication", pages 62-69.
- VOET; VOET: 'Biochemistry', VHC VERLAGSGESELLSCHAFT

## Description

This patent summarizes the discovery of the protein code and the therapeutic and diagnostic use of this new biological language for different areas of medicine. This patent claims for the use of new algorithms to identify signal oligopeptides.

The discovery of the genetic code four decades ago (Watson JD, Crick FHC. 1953. Nature 171:737) defined the principles by which genes encode for proteins. The protein code, however, the biological language by which proteins act and interact has remained obscure.

It is known from US-Patent No. 4.554.101 (Hopp) to synthesize a peptide comprising a sequence of at least six amino acid residues corresponding to the antigenic or allergenic determinant on an antigenic or allergenic protein on the base of hydrophilicity.

It is further known from Rath, Cationic-anionic and Anionic-cationic Oligopeptides in Apoprotein (a) and other Proteins as Modulators of Protein Action and of biological Communication (journal of applied nutrition, volume 44, no. 3 and 4, 1992), that assumes that cationic-anionic and anionic-cationic oligopeptides may play a role in conformation within a given protein molecule and that structural motives may be responsible for selective exchange of biological information.

Now the protein code is discovered Rath, M. 1994. J Appl. Nutr. (in Press). The interaction of hormones and other ligands with their respective receptors, of enzymes with protein substrates, of adhesive proteins with integrins, and of antibodies with antigens as well as other protein actions and interactions are determined by the same structure/function principles and the same biological language. For a very limited number of proteins the hydrophobic interior of the protein forms the active centre which also determines their function. In the vast majority of proteins, however, biological activity is primarily mediated via specific surface structures which mediate their specific functions. The structure/function principles determining these protein actions are discovered and they are hereafter referred to as the protein code.

Like the human language the protein code consists of letters words and sentences. The letters (amino acids) and sentences (complete three dimensional protein) had been known before. The new discovery are the protein words or verbs. These protein verbs are represented by signal oligopeptides which are localised on the surface of the protein and are represented by the hydrophilicity maxima of the protein. These signal oligopeptides are enriched in charged amino acids in a versatile arrangement with neutral spacer amino acids. The specific signal character of these oligopeptides is determined by a characteristic combination of conformation and charge within the same signal sequence.

Like in the human language the whole sentence (complete three dimensional protein) is needed to determine the specific and complete action of any given protein. In the human language eliminating or changing the verb of a sentence renders the whole sentence meaningless. Similarly, blocking the protein code verbs (signal oligopeptides) can be therapeutically used to block the undesired action or interaction of an entire protein.

The discovery of the protein code provides the rationale for deciphering the communication code of diseases. Infectious diseases, cancer, cardiovascular and other diseases develop by means of one or more pathogenicity-mediating proteins. Blocking the signal oligopeptides of these proteins allows the specific therapeutic interception of a pathological communication and thereby blocks disease propagation.

Synthetic analogues to signal oligopeptides can be used therapeutically in several ways.

Synthetic analogues to signal oligopeptides can be used as competitive inhibitors of pathological communication.

Conventional drug therapy is frequently compromised by an unknown therapeutic mechanism and by a wide range of side-effects and considerable toxicity.

There is a need for a method of therapy that allows for the interception of pathological interactions with maximum effectiveness.

There is a further need for a method of therapy that enables maximum therapeutic specificity based on the precise structure/function relation of specific oligopeptide signals.

The invention discloses the use of a method of producing therapeutic peptides in the prevention and treatment of human disease which are caused by one ore more proteins. This method of peptide therapy, comprises identifying the protein responsible for causing the human disease, identifying one or more signal oligopeptide sequences within the structure of the disease causing protein, the one or more signal oligopeptides representing the amino-acid sequence of maximum hydrophilicity and synthesising one or more oligopeptides having amino-acid sequences corresponding to the amino-acid sequences of the signal oligopeptides of maximum hydrophilicity.

In an alternative embodiment the method comprises identifying the one or more signal oligopeptides representing the amino-acid sequence of maximum surface probability as defined as that portion of the amino-acid sequence that has a higher probability of being on the surface of the protein.

In an alternative embodiment the method further comprises a method of identifying one or more signal oligopeptide sequences which represent the amino-acid sequence of maximum electrical charge of the amino-acids in the disease causing protein.

The above-mentioned methods can be used for the production of therapeutic agents to function as direct competitive inhibitors to specifically prevent or reduce the metabolic action or interaction of a selected protein by partially or completely blocking the specific signal sequences.

The above-mentioned methods of producing therapeutic agents analogues to the specific oligopeptides number 1 to 360 of the attached sequence listing which vary from the sequences given by omitting the amino residuals of the N-terminal end, at the C-terminal end or both ends or by substituting one or more of the amino-acid residuals within the given sequence without consideration of charge and polarity of the substituting residual, by substituting residuals with similar charge and/or polarity or by omitting one or more amino-acids within the given sequence or finally by a combination of two or more of the above modifications.

In the human language eliminating or changing the verb of a sentence renders the whole sentence meaningless. Similarly, blocking the protein code verbs (signal oligopeptide sequences) can be therapeutically used to block the undesired action or interaction of an entire protein. If the verb in any sentence is altered, i.e. change eating to walking, the entire meaning of the sentence changes, or the sentence is rendered unintelligible. These verbs of the protein code, referred to as signal oligopeptides, determine the very specific function of a protein. Similarly, if a signal oligopeptide is altered (or blocked) in any given protein, either that protein's function changes, or the protein is rendered functionless. Interference with signal oligopeptides, these verbs of the protein code sentences, can lead to substantial modification or loss of the biological message of a protein. This modification or loss of the biological message of the protein can be used in peptide therapy to the advantage of a patient.

The specific action of these oligopeptides is determined by a characteristic combination of shape and electrical charge (both anionic and cationic) within the same signal sequence. Protein information transfer and protein interaction is dependent on the accessibility of the signal oligopeptide sequence. Therefore, in the majority of proteins, the sequence signals are localised on the surface of the protein. Signal oligopeptides are enriched with charged amino acids such as cationic amino acid residues arginine and lysine and/or the anionic amino acid residues glutamate and aspartate. Sometimes, these signal oligopeptides are in a versatile arrangement with neutral spacer amino acids. Therefore, signal oligopeptide sequences are generally represented by the regions of maximum hydrophilicity on the surface of the protein molecule.

A new type of signal is represented by oligopeptides which obtain their characteristic conformation or shape by a specific arrangement of oppositely charged amino acid residues within this oligopeptide sequence. These residues with opposite charge can attract each other thereby modulating a characteristic folding of this signal sequence. For example, in the signal sequence RGD the cationic residue arginine and the amino residue aspartate attract each other leading to a characteristic folding of this tripeptide around the 'spacer' residue glycine.

The specific metabolic function of a protein is dependent on the specificity of its biological signal oligopeptide. The signal character of a specific signal oligopeptide is determined by a characteristic combination of electrical charge with structural conformation. Within a protein, RGD and analogous tripeptides can serve as strong primary anchors while the specific biological message is mediated by additional longer and more complex signal oligopeptides.

Synthetic analogues of signal oligopeptide sequences are used therapeutically in several ways. First, synthetic analogues of signal oligopeptide sequences can be used as competitive inhibitors of pathological communication.

The method of this invention using synthetic analogues is based on the discovery of the primary structural principles determining immunogenicity. The discrimination between self and non-self between species of animals and humans is primarily based on amino acid residue substitutions or other residue variations within the signal oligopeptide sequences of a protein. By making use of this discovery effective therapeutic signal oligopeptides can be rapidly produced in the following way: signal oligopeptides of a given protein in one species of animals are the antigenicity determinants of this protein in another species of animals. To block the action of a pathogenicity-mediating or disease causing protein in the treatment of a human disease the synthetic signal oligopeptides are designed by copying corresponding amino acid signal sequences from another species. A glucagon signal oligopeptide for the treatment of diabetic patients would be based on glucagon signal sequences from rabbits, sheep, mice or other species. A titration of the therapeutic efficiency is possible using the evolutionary chain method described below. The greater the genetic and evolutionary distance of the selected animal species to humans the greater its antigenicity and, consequently, the greater its therapeutic efficiency.

Furthermore, signal oligopeptides of a protein are identical with the potential antigenic determinants. Antibodies and other mediators of immune response are interceptors of specific biological communication. Signal oligopeptides as promoters of differentiated protein communication and immune response mediators as interceptors form sophisticated network of biological communication. Therefore, decoding the physiological aspects of this communication network will lead to a precise understanding of millions of metabolic interactions-including the principles for development and differentiation of the body, which will lead to the therapeutic control of many diseases and eventually their eradication as causes of human mortality.

Turning now to the figures the invention is described in detail.

**Table 1;** shows the various amino acids found in proteins and their hydrophilicity and surface probability values.

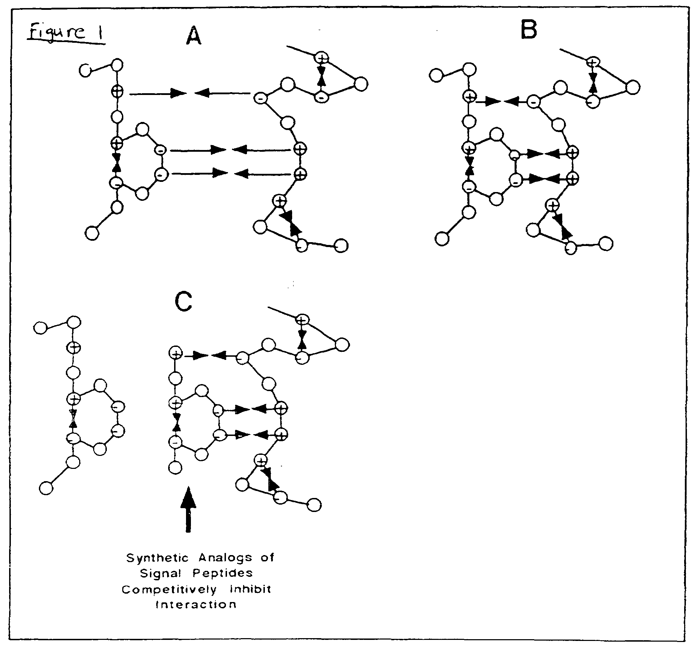

**Fig.1A and 1B** show several of the protein code interaction principles including legibility, accessibility, variability, and specificity.
**Fig. 1C shows the oppositely** charged amino acids can attract each other thereby enhancing conformational specificity.

Figures 1 A and 1 B show principles upon which the protein code functions. Within the amino acid sequence of a disease causing protein one or more signal oligopeptides represent the "verbs" of the protein code which determine the specific action and interaction of that protein. This is referred to as the legibility of the protein. The signal oligopeptides of a protein are enriched in electrically charged amino acids (either cationic or anionic) and represent a segment of maximum hydrophilicity within the protein sequence. An infinite number of possible combinations between amino acids with different charges as well as neutral residues provide the variability for differentiated metabolic communication. The specificity of a signal sequence is the result of a characteristic combination of charge distribution and structural conformation within the signal oligopeptide sequence. Figure 1 B shows the oppositely charged amino acids attracting each other thereby enhancing conformational specificity. Signal oligopeptides mediate specific information transfer to their metabolic counterparts. Substitution, deletion or other amino acid residue variations within the signal sequence of a protein enable differentiation between 'self and non-self'. Signal sequences are the antigenic epitopes of a protein and are responsible for potential immune responses when exposed to other organisms. Direct Peptide Interception Therapy (PIT) can be used to intercept undesired or pathological communication and thereby block the disease. Figure 1 C shows direct PIT synthetic analogues of signal oligopeptides used to competitively inhibit the interaction of proteins.

In contrast, peptide therapy is based on the principle that at some point, the genetic code must be translated into proteins that then interact with cells, and that health or disease is ultimately decided at the level of proteins. The identification and therapeutic use of the key oligopeptides within a selected protein is the most direct, specific, effective, as well as the safest and most affordable way for the prevention or treatment of the disease. Compared to gene therapy, the application of peptide therapy will shorten the time for development and treatment for many human diseases. Therefore, peptide therapy as described below has the following advantages:

Peptide therapy is a highly effective form of treatment. The discovery of the peptide code provides the rationale for deciphering the communication code of proteins in health and disease. This discovery is used therapeutically to intercept pathological interactions of human disease with maximum effectiveness.

Peptide therapy also enables maximum therapeutic specificity. Based on the precise understanding of the structure/function relation of specific protein signals, peptide therapy allows therapeutic targeting with unprecedented specificity.

Furthermore, peptide therapy is extremely safe. The use of synthetic analogues to physiological compounds essentially eliminates the problem of toxicity. Possible undesired biological side-effects are controllable by optimising the length and composition of the therapeutic peptide.

Time and expenses for the development of therapeutic peptides is a fraction of conventional therapeutic research and development. Identification of potential therapeutic peptides takes minutes; in vitro screening of potential peptides is a matter of weeks; animal studies should provide first in vivo results within a few months. Most importantly, the unprecedented specificity and safety background of peptide therapy will allow clinical studies without delay.

Infectious diseases, cancer, cardiovascular and other human diseases develop by means of one or more pathogenicity-mediating proteins, or disease causing proteins. Blocking the action of these proteins allows the specific therapeutic interception of a pathological communication, thereby blocking disease propagation.

Signal tetrapeptides, pentapeptides, hexapeptides and longer peptides represent primary candidates for specific peptide therapy. Shorter peptides, such as the tripeptide RGD, are less specific and ubiquitous side effects limit their broad therapeutic use.

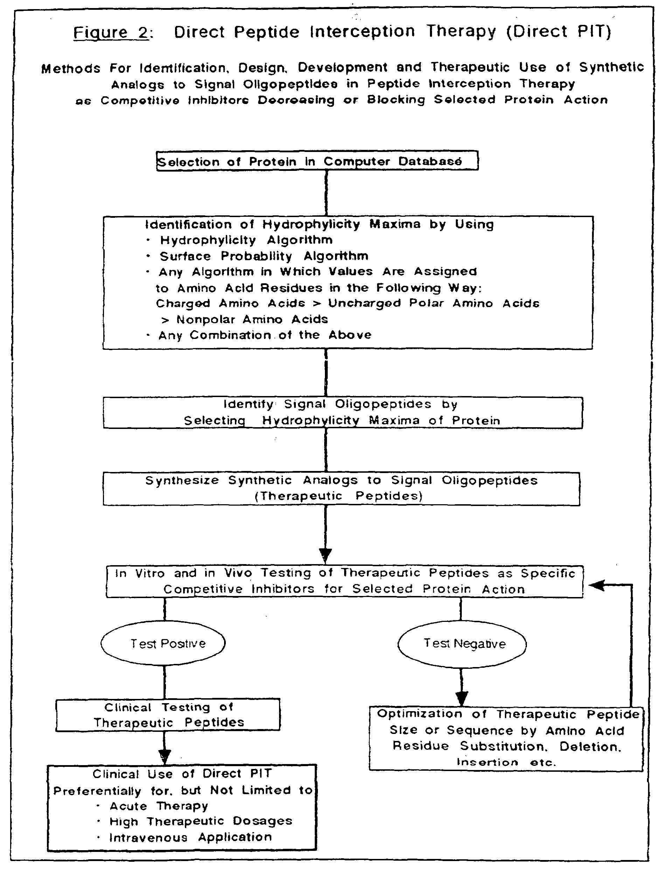

Figure 2 shows the fundamentals of Peptide Interception Therapy (PIT). Proteins are essential carriers of specific metabolic information. Moreover, proteins are frequently mobile which makes them ideal and versatile communication molecules. This figure illustrates several key elements of direct PIT in peptide therapy. In conventional gene therapy, the entire three dimensional protein structure (protein sentence) is required to counter the effects of the disease causing protein. However, in direct peptide interception therapy, only the signal oligopeptide sequence ("verb") has to be blocked. Figure 2 also shows methods for identification, design, development and therapeutic use of synthetic analogues to signal oligopeptides in Peptide Interception Therapy as direct competitive inhibitors of selected protein actions.

As further described in detail below, peptide Interception Therapy (PIT) is used in a principal way: direct PIT, which uses synthetic analogues of signal oligopeptides as competitive inhibitors of pathological or undesired metabolic interaction

As indicated above, Direct Peptide Interception Therapy (Direct PIT) uses synthetic analogues of signal oligopeptides as direct competitive inhibitors for undesired protein communication. Direct blocking of pathogenicity mediating protein communication leads to the control of the related disease or clinical condition. This therapeutic approach is preferentially used in acute conditions, e.g. antithrombotic or fibrinolytic therapy.

The protein code is a key for individual development within a species as well as for the evolutionary diversification of species. The effectiveness of signal oligopeptides to mediate specific biological messages were the ultimate criterion for the evolutionary advantage of a protein and, thus, for the evolutionary survival of the gene encoding for it. Genetic mutations leading to the substitution of one or more amino acid residues within a signal oligopeptide sequence were an economic and therefore frequent mechanism to modulate and differentiate protein action and thereby promoting evolutionary diversification.

Once the protein sequence's oligopeptide is identified, synthetic analogues are produced to match the sequence of the oligopeptide. If the synthetic analogues are not effective as feedback regulators, the analogues are optimised to produce the desired effect using peptide size alteration by amino acid residue substitution, deletion, insertion, or any combination thereof.

Antigenicity required for effective PIT therapy can also be stimulated in conventional ways, e.g. by omitting one or more amino acid residues at the N-terminal end of any given sequence (start of sequence), by omitting one or more amino acid residues at the C-terminal end of any given sequence (end of terminal), by omitting one or more amino acid residues at the N-terminal and the C-terminal end of any given sequence, by substituting one or more of the amino acid residues within any given sequence without consideration of charge and polarity of the substitution residue, by substituting one or more of the amino acid residues within any given sequences with amino acid residues with similar charge and/or polarity, by omitting one or more amino acid residues within any given sequence, by coupling the therapeutic peptides to defined haptens or other immunogenic compounds enhancing non-self' recognition, or a combination of two or more of the mentioned methods.

Oligopeptide Sequences can be used in Therapeutic Applications of for infectious diseases. The most immediate application of peptide therapy is the prevention and treatment of infectious diseases. Every stage of any type of infectious disease is controlled by proteins which mediate adhesion, invasion and other mechanisms of pathogenicity. Effective therapeutic interception of biological signals within these pathogenicity-mediating proteins must lead to the control of the infection itself. As discussed above, for diseases mediated by xenologous proteins (infectious diseases) a signal sequence is chosen from xenologous protein (toxin) as basis for the peptides in humans. The peptides are then used for the prevention and treatment of the infectious disease.

Another area with immediate applications for peptide therapy is the treatment of neoplastic diseases. Invasive growth and metastatic spread of any type of cancer is mediated by certain proteins and their signal sequences. Synthetic analogues to the signal sequences of these proteins should be developed as an effective therapy for different forms of cancer in their early stage as well as their invasive and metastatic stages.

A novel therapeutic area for peptide therapy is the treatment of metabolic disorders. The potential of peptide therapy is exemplified here for the treatment of diabetes and hypertension, which are described in detail in the Examples below:
1. Therefore, synthetic analogues to human signal oligopeptide sequences are used for,
   a. as therapeutic agents for direct competitive inhibition of selected protein interaction,
   b. as therapeutic agents in feedback regulation with aim to decrease the synthesis rate of the selected protein,
2. Synthetic analogues to protein signal sequences from other species are used;
   a. as therapeutic agents in the prevention and treatment of human diseases, stimulating a specific immune response which blocks or decreases the action of the selected protein in the human body.
   b. As therapeutic agents according to sections 1a and 1b above in the treatment of diseases in the respective animal species.

Using the method of the invention as described above aids in the identification of potential therapeutic peptides in minutes; in vitro screening of potential peptides is a matter of weeks; and animal studies should provide first in vivo results within a few months.

### Example No. 1

Conventional diabetic therapy aims at an increased availability of insulin. Peptide therapy allows a novel and alternative approach by inhibiting the action of glucagon, the insulin antagonist. Amino acid sequence selection and therapeutic design of peptide vaccines for Indirect Peptide Interception Therapy, exemplified for the development of glucagon vaccines in clinical therapy of diabetes mellitus is described here.

The inhibition of glucagon is accomplished by using the therapeutic peptides analogous to the glucagon signal sequence for direct competitive inhibition (F2). First, the signal oligopeptide is identified from a human Glucagon Precursor sequence. Using the available data for Glucagon Precursor sequences in different species, a corresponding signal oligopeptide is identified to the human oligopeptide. Using the evolutionary tree, the relative distance of the available Glucagon Sequence to the human sequence is determined. The evolutionary distance is positively correlated with degree of amino acid variation and therefore, with the antigenicity of the selected protein.

The therapeutic peptide sequence is selected among the corresponding sequences according to the following criteria. If a moderate therapeutic immune response is desired, then the therapeutic signal sequence is preferably derived from species that are genetically close to humans (e.g. mammals). If a strong therapeutic response is desired, then the therapeutic peptides are designed from species that are genetically more distant to humans (e.g. Fish, Yeast).

### Example No. 2

The renin angiotensin System and particularly the angiotensin-converting enzyme (ACE) are a continuous focus of antihypertensive drug development. Renin, angiotensin I and II as well as ACE are also promising targets for peptide therapy. Therapeutic use of synthetic analogues to the signal sequences of any of these proteins will lead to decreased blood pressure. Since in most cases hypertension is a chronic condition the therapeutic use of renin, angiotensin or ACE signal peptide vaccines is a preferable method of treatment.

### Example No. 3

The following proteins' signal sequences are derived from the method of this invention and are used for diagnostic as well as therapeutic purposes as described above.

| | |
|---|---|
| Farnesyl Synthetase | Sequence Id Nos. 1-41 |
| Hydroxy-Methyl-Glutaryl Coenzyme A Reductase | Sequence Id Nos. 42 -163 |
| Gonadoliberin Precursor | Sequence Id Nos. 164 - 172 |
| Plasminogen Activator Inhibitor 1 | Sequence Id Nos. 173 - 194 |
| Plasminogen Activator Inhibitor 2 | Sequence Id Nos. 195 - 238 |
| Herpes Virus 1 (HSV 1) Glycoprotein B | Sequence Id Nos. 239 - 244 |
| Herpes Virus 2 (HSV 23, 2H) Glycoprotein B | Sequence Id Nos. 245 - 251 |
| Treponema Pallidum Membrane Protein (TMPA) | Sequence Id Nos. 252 - 262 |
| Islet Amyloid Polypeptide | Sequence Id Nos. 263 - 268 |
| Collagenase (Fibroblast MMP 1) | Sequence Id Nos. 269 - 280 |
| Schistosoma Elastase Precursor | Sequence Id Nos. 281 - 284 |
| Schistosomin | Sequence Id Nos. 285 - 287 |
| Apolipoprotein (a) Human | Sequence Id Nos. 288 - 289 |
| Apolipoprotein (a) Rhesus | Sequence Id Nos. 290 - 295 |
| Hepatitis Delta Antigen | Sequence Id Nos. 296 - 298 |
| Rev Protein HIV, SIV, VILV, OMVVS | Sequence Id Nos. 299 - 348 |
| Corticotropin Releasing Factor Binding Protein | Sequence Id Nos. 349 - 360 |

**The following specific sequences are claimed in this patent:**
**FARNESYL SYNTHETASE**
   INFORMATION FOR SEQUENCE ID NO 1
      (A) LENGTH:
      (B) TYPE: amino acid
      (D) TOPOLOGY: linear
         -D-V-Y-A-Q-E-K-O-D-
   INFORMATION FOR SEQUENCE ID NO 2
      (A) LENGTH:
      (B) TYPE: amino acid
      (D) TOPOLOGY: linear
         -D-V-H-N-Q-E-K-Q-N-
   INFORMATION FOR SEQUENCE ID NO 3
      (A) LENGTH:
      (B) TYPE: amino acid
      (D) TOPOLOGY: linear
         -E-I-R-R-E-R-
   INFORMATION FOR SEQUENCE ID NO 4
      (A) LENGTH:
      (B) TYPE amino acid
      (D) TOPOLOGY: linear
         -E-D-E-M-G-H-P-E-I-G-D-
   INFORMATION FOR SEQUENCE ID NO 5
      (A) LENGTH:
      (B) TYPE: amino acid
      (D) TOPOLOGY: linear
         -E-D-E-L-G-H-P-E-K-G-D-
   INFORMATION FOR SEQUENCE ID NO 6
      (A) LENGTH:
      (B) TYPE: amino acid
      (D) TOPOLOGY: linear
         -A-S-L-L-A-Y-G-M-P-K-E-
   INFORMATION FOR SEQUENCE ID NO 7
      (A) LENGTH:
      (B) TYPE: amino acid
      (D) TOPOLOGY: linear
         -E-P-R-K-Q-D-A-D-
   INFORMATION FOR SEQUENCE ID NO 8
      (A) LENGTH:
      (B) TYPE: amino acid
      (D) TOPOLOGY: linear
         -E-P-R-K-O-D-A-E-
   INFORMATION FOR SEQUENCE ID NO 9
      (A) LENGTH:
      (B) TYPE: amino acid
      (D) TOPOLOGY: linear
         -N-K-T-V-E-Q-G-L-Q-E-E-
   INFORMATION FOR SEQUENCE ID NO 10
      (A) LENGTH:
      (B) TYPE: amino acid
      (D) TOPOLOGY: linear
         -D-D-I-M-D-S-S-L-T-R-R-
   INFORMATION FOR SEQUENCE ID NO 11
      (A) LENGTH:
      (B) TYPE: amino acid
      (D) TOPOLOGY: linear
         -D-D-I-M-D-S-S-Y-T-R-R-
   INFORMATION FOR SEQUENCE ID NO 12
      (A) LENGTH:
      (B) TYPE: amino acid
      (D) TOPOLOGY: linear
         -D-D-M-M-D-K-S-I-T-R-R-
   INFORMATION FOR SEQUENCE ID NO 13
      (A) LENGTH:
      (B) TYPE: amino acid
      (D) TOPOLOGY: linear
         -K-L-Y-C-R-E-Q-P-Y-Y-
   INFORMATION FOR SEQUENCE ID NO 14
      (A) LENGTH:
      (B) TYPE: amino acid
      (D) TOPOLOGY: linear
         -K-S-H-F-R-N-E-K-Y-Y-
   INFORMATION FOR SEQUENCE ID NO 15
      (A) LENGTH:
      (B) TYPE: amino acid
      (D) TOPOLOGY: linear
         -R-F-T-E-K-R-Y-K-
   INFORMATION FOR SEQUENCE ID NO 16
      (A) LENGTH:
      (B) TYPE: amino acid
      (D) TOPOLOGY: linear
         -R-Y-T-E-K-R-Y-K-
   INFORMATION FOR SEQUENCE ID NO 17
      (A) LENGTH:
      (B) TYPE: amino acid
      (D) TOPOLOGY: linear
         -K-F-S-L-K-K-H-S-
   INFORMATION FOR SEQUENCE ID NO 18
      (A) LENGTH:
      (B) TYPE: amino acid
      (D) TOPOLOGY: linear
         -K-V-D-L-S-K-F-S-L-K-K-
   INFORMATION FOR SEQUENCE ID NO 19
      (A) LENGTH:
      (B) TYPE: amino acid
      (D) TOPOLOGY: linear
         -K-K-H-S-F-I-V-T-F-K-T-
   INFORMATION FOR SEQUENCE ID NO 20
      (A) LENGTH:
      (B) TYPE: amino acid
      (D) TOPOLOGY: linear
         -D-G-E-K-E-H-A-N-A-K-K-
   INFORMATION FOR SEQUENCE lD NO 21
      (A) LENGTH:
      (B) TYPE : amino acid
      (D) TOPOLOGY: linear
         -D-G-E-K-E-H-A-N-A-L-K-
   INFORMATION FOR SEQUENCE ID NO 22
      (A) LENGTH:
      (B) TYPE: amino acid
      (C) TOPOLOGY: linear
         -T-G-E-K-D-L-K-O-A-R-D-
   INFORMATION FOR SEQUENCE ID NO 23
      (A) LENGTH:
      (B) TYPE: amino acid
      (D) TOPOLOGY: linear
         -K-I-G-T-D-I-Q-D-N-K-
   INFORMATION FOR SEQUENCE ID NO 24
      (A) LENGTH:
      (B) TYPE: amino acid
      (D) TOPOLOGY: linear
         -K-V-G-T-D-I-Q-D-N-K-
   INFORMATION FOR SEQUENCE ID NO 25
      (A) LENGTH:
      (B) TYPE: amino acid
      (D) TOPOLOGY: linear
         -Q-R-A-T-P-E-Q-Y-
   INFORMATION FOR SEQUENCE ID NO 26
      (A) LENGTH:
      (B) TYPE: amino acid
      (D) TOPOLOGY: linear
         -L-R-A-T-P-Q-Q-R-
   INFORMATION FOR SEQUENCE ID NO 27
      (A) LENGTH:
      (B) TYPE: ami no acid
      (D) TOPOLOGY: linear
         -E-L-A-S-A-E-Q-R-
   INFORMATION FOR SEQUENCE ID NO 28
      (A) LENGTH:
      (B) TYPE: amino acid
      (D) TOPOLOGY: linear
         -K-E-N-Y-G-Q-K-E-A-E-K-
   INFORMATION FOR SEQUENCE ID NO 29
      (A) LENGTH:
      (B) TYPE: amino acid
      (D) TOPOLOGY: linear
         -E-E-N-Y-G-Q-K-D-P-E-K-
   INFORMATION FOR SEQUENCE ID NO 30
      (A) LENGTH:
      (B) TYPE: amino acid
      (D) TOPOLOGY: linear
         -D-E-N-Y-G-K-K-D-S-
   INFORMATION FOR SEQUENCE ID NO 31
      (A) LENGTH:
      (B) TYPE: amino acid
      (D) TOPOLOGY: linear
         -R-V-K-A-L-Y-E-E-L-
   INFORMATION FOR SEQUENCE ID NO 32
      (A) LENGTH:
      (B) TYPE: amino acid
      (D) TOPOLOGY: linear
         -K-C-K-K-I-F-N-D-L-
   INFORMATION FOR SEQUENCE ID NO 33
      (A) LENGTH:
      (B) TYPE: amino acid
      (D) TOPOLOGY: linear
         -K-E-A-E-K-V-A-R-V-K-
   INFORMATION FOR SEQUENCE ID NO 34
      (A) LENGTH:
      (B) TYPE: amino acid
      (D) TOPOLOGY: linear
         -K-D-S-V-A-E-A-K-C-K-
   INFORMATION FOR SEQUENCE ID NO 35
      (A) LENGTH:
      (B) TYPE: amino acid
      (D) TOPOLOGY: linear
         -K-D-P-E-K-V-A-R-
   INFORMATION FOR SEQUENCE ID NO 36
      (A) LENGTH:
      (B) TYPE: amino acid
      (D) TOPOLOGY: linear
         -Q-Y-E-E-D-S-Y-S-H-
   INFORMATION FOR SEQUENCE ID NO 37
      (A) LENGTH:
      (B) TYPE: amino acid
      (D) TOPOLOGY: linear
         -K-Y-E-E-D-S-Y-N-R-
   INFORMATION FOR SEQUENCE ID NO 38
      (A) LENGTH:
      (B) TYPE: amino acid
      (D) TOPOLOGY: linear
         -E-Y-E-E-S-I-A-K-D-
   INFORMATION FOR SEQUENCE ID NO 39
      (A) LENGTH:
      (B) TYPE: amino acid
      (D) TOPOLOGY: linear
         -R-K-I-Y-K-R-R-K-
   INFORMATION FOR SEQUENCE ID NO 40
      (A) LENGTH:
      (B) TYPE: ammo acid
      (D) TOPOLOGY: linear
         -N-K-I-Y-K-R-R-K-
   INFORMATION FOR SEQUENCE ID NO 41
      (A) LENGTH:
      (B) TYPE: amino acid
      (D) TOPOLOGY: linear
         -N-K-V-Y-K-R-S-K-
**HYDROXY-METHYL-GLUTARYL COENZYME A REDUCTASE**
   INFORMATION FOR SEQUENCE ID NO 42
      (A) LENGTH:
      (B) TYPE: amino acid
      (D) TOPOLOGY: linear
         -S-Q-D-E-V-R-E-N-
   INFORMATION FOR SEQUENCE ID NO 43
      (A) LENGTH:
      (B) TYPE: amino acid
      (D) TOPOLOGY: linear
         -T-Q-N-E-V-V-D-N-
   INFORMATION FOR SEQUENCE ID NO 44
      (A) LENGTH:
      (B) TYPE: amino acid
      (D) TOPOLOGY: linear
         -E-L-S-R-E-S-R-E-G-R-
   INFORMATION FOR SEQUENCE ID NO 45
      (A) LENGTH:
      (B) TYPE: amino acid
      (D) TOPOLOGY: linear
         -E-L-S-N-S-N-K-Y-G-R-
   INFORMATION FOR SEQUENCE ID NO 46
      (A) LENGTH:
      (B) TYPE: amino acid
      (D) TOPOLOGY: linear
         -R-V-L-E-E-E-E-N-K-
   INFORMATION FOR SEQUENCE ID NO 47
      (A) LENGTH
      (B) TYPE: amino acid
      (D) TOPOLOGY: linear
         -E-V-L-E-E-E-E-D-R-K-
   INFORMATION FOR SEQUENCE ID NO 48
      (A) LENGTH
      (B) TYPE: amino acid
      (D) TOPOLOGY: linear
         -K-G-V-S-S-S-T-R-K-
   INFORMATION FOR SEQUENCE ID NO 49
      (A) LENGTH:
      (B) TYPE: amino acid
      (D) TOPOLOGY: linear
         -S-V-L-E-E-E-E-D-N-K-
   INFORMATION FOR SEQUENCE ID NO 50
      (A) LENGTH:
      (B) TYPE: amino acid
      (D) TOPOLOGY: linear
         -D-E-N-V-S-K-R-I-E-P-
   INFORMATION FOR SEQUENCE ID NO 51
      (A) LENGTH:
      (B) TYPE: amino acid
      (D) TOPOLOGY: linear
         -D-D-M-M-P-K-R-V-E-
   INFORMATION FOR SEQUENCE ID NO 52
      (A) LENGTH
      (B) TYPE: amino acid
      (D) TOPOLOGY: linear
         -D-F-T-M-P-L-W-E-F-
   INFORMATION FOR SEQUENCE ID NO 53
      (A) LENGTH
      (B) TYPE: amino acid
      (D) TOPOLOGY: linear
         -K-K-V-P-D-N-C-C-R-R-E-
   INFORMATION FOR SEQUENCE ID NO 54
      (A) LENGTH
      (B) TYPE: amino acid
      (D) TOPOLOGY: linear
         -K-K-A-P-D-N-C-C-R-R-E-
   INFORMATION FOR SEQUENCE ID NO 55
      (A) LENGTH:
      (B) TYPE: amino acid
      (D) TOPOLOGY: linear
         -K-K-Q-I-E-S-C-C-R-R-E-
   INFORMATION FOR SEQUENCE ID NO 56
      (A) LENGTH:
      (B) TYPE: amino acid
      (D) TOPOLOGY: linear
         -Q-K-C-D-S-V-E-E-
   INFORMATION FOR SEQUENCE ID NO 57
      (A) LENGTH:
      (B) TYPE: amino acid
      (D) TOPOLOGY: linear
         -E-K-L-S-S-V-E-E-
   INFORMATION FOR SEQUENCE ID NO 58
      (A) LENGTH
      (B) TYPE: amino acid
      (D) TOPOLOGY: linear
         -R-V-S-T-T-E-E-
   INFORMATION FOR SEQUENCE ID NO 59
      (A) LENGTH
      (B) TYPE: amino acid
      (D) TOPOLOGY: linear
         -E-T-L-I-S-D-Q-
   INFORMATION FOR SEQUENCE ID NO 60
      (A) LENGTH:
      (B) TYPE: amino acid
      (D) TOPOLOGY: linear
         -E-E-T-G-I-N-R-E-R-K-V-E-
   INFORMATION FOR SEQUENCE ID NO 61
      (A) LENGTH:
      (B) TYPE: amino acid
      (D) TOPOLOGY: linear
         -E-E-P-G-V-S-Q-D-R-K-V-E-
   INFORMATION FOR SEQUENCE ID NO 62
      (A) LENGTH:
      (B) TYPE: amino acid
      (D) TOPOLOGY: linear
         -E-A-S-S-K-E-E-T-E-A-
   INFORMATION FOR SEQUENCE ID NO 63
      (A) LENGTH:
      (B) TYPE: amino acid
      (D) TOPOLOGY: linear
         -T-D-F-V-T-A-S-F-R-N-
   INFORMATION FOR SEQUENCE ID NO 64
      (A) LENGTH
      (B) TYPE: amino acid
      (D) TOPOLOGY: linear
         -E-P-E-I-E-L-P-R-E-P-R-P-N-E-E-
   INFORMATION FOR SEQUENCE ID NO 65
      (A) LENGTH:
      (B) TYPE: amino acid
      (D) TOPOLOGY: linear
         -E-L-E-I-E-L-P-S-E-P-R-P-N-E-E-
   INFORMATION FOR SEQUENCE ID NO 66
      (A) LENGTH
      (B) TYPE: amino acid
      (D) TOPOLOGY: linear
         -N-T-M-F-D-L-P-E-E-P-R-P-L-D-E-
   INFORMATION FOR SEQUENCE ID NO 67
      (A) LENGTH:
      (B) TYPE: amino acid
      (D) TOPOLOGY: linear
         -E-V-E-W-V-L-E-T-E-L-K-A-P-R-P-
   INFORMATION FOR SEQUENCE ID NO 68
      (A) LENGTH:
      (B) TYPE: amino acid
      (D) TOPOLOGY: linear
         -R-E-P-R-P-N-E-E-C-
   INFORMATION FOR SEQUENCE ID NO 69
      (A) LENGTH
      (B) TYPE: amino acid
      (D) TOPOLOGY: linear
         -E-L-K-A-P-R-P-M-
   INFORMATION FOR SEQUENCE ID NO 70
      (A) LENGTH
      (B) TYPE: amino acid
      (D) TOPOLOGY: linear
         -E-E-P-R-P-L-D-E-C-
   INFORMATION FOR SEQUENCE ID NO 71
      (A) LENGTH:
      (B) TYPE: amino acid
      (D) TOPOLOGY: linear
         -H-E-R-G-U-S-I-R-R-Q-L-L-S-K-K-
   INFORMATION FOR SEQUENCE ID NO 72
      (A) LENGTH:
      (B) TYPE: amino acid
      (D) TOPOLOGY: linear
         -P-R-E-G-V-A-I-R-R-Q-M-L-S-D-K-
   INFORMATION FOR SEQUENCE ID NO 73
      (A) LENGTH:
      (B) TYPE: amino acid
      (D) TOPOLOGY: linear
         -P-E-R-G-V-A-V-R-R-Q-I-I-S-K-
   INFORMATION FOR SEQUENCE ID NO 74
      (A) LENGTH:
      (B) TYPE: amino acid
      (D) TOPOLOGY: linear
         -P-F-R-A-V-E-L-R-R-L-D-L-
   INFORMATION FOR SEQUENCE ID NO 75
      (A) LENGTH:
      (B) TYPE: amino acid
      (D) TOPOLOGY: linear
         -L-Q-Y-L-P-Y-R-D-Y-N-
   INFORMATION FOR SEQUENCE ID NO 76
      (A) LENGTH:
      (B) TYPE: amino acid
      (D) TOPOLOGY: linear
         -I-E-R-I-P-Y-K-D-Y-D-
   INFORMATION FOR SEQUENCE ID NO 77
      (A) LENGTH:
      (B) TYPE: amino acid
      (D) TOPOLOGY: linear
         -L-Q-S-L-P-Y-K-N-Y-N-
   INFORMATION FOR SEQUENCE ID NO 78
      (A) LENGTH:
      (B) TYPE: amino acid
      (D) TOPOLOGY: linear
         -L-E-K-L-P-Y-A-S-Y-D-
   INFORMATION FOR SEQUENCE ID NO 79
      (A) LENGTH:
      (B) TYPE: amino acid
      (D) TOPOLOGY: linear
         -C-L-D-E-K-E-F-Q-
   INFORMATION FOR SEQUENCE ID NO 80
      (A) LENGTH:
      (B) TYPE : amino acid
      (D) TOPOLOGY: linear
         -C-L-D-G-K-E-Y-Q-
   INFORMATION FOR SEQUENCE ID NO 81
      (A) LENGTH:
      (B) TYPE: amino acid
      (D) TOPOLOGY: linear
         -L-L-N-N-K-E-Y-Q-
   INFORMATION FOR SEQUENCE ID NO 82
      (A) LENGTH:
      (B) TYPE: amino acid
      (D) TOPOLOGY: linear
         -L-L-D-G-Q-E-F-Q-
   INFORMATION FOR SEQUENCE ID NO 83
      (A) LENGTH:
      (B) TYPE: amino acid
      (D) TOPOLOGY: linear
         -L-L-D-G-R-S-H-Y-
   INFORMATION FOR SEQUENCE ID NO 84
      (A) LENGTH:
      (B) TYPE: amino acid
      (D) TOPOLOGY: linear
         -R-L-P-R-A-C-D-S-A-E-V-K-
   INFORMATION FOR SEQUENCE ID NO 85
      (A) LENGTH:
      (B) TYPE: amino acid
      (D) TOPOLOGY: linear
         -R-L-P-T-A-C-D-A-A-E-V-K-
   INFORMATION FOR SEQUENCE ID NO 86
      (A) LENGTH:
      (B) TYPE: amino acid
      (D) TOPOLOGY: linear
         -R-L-P-S-A-Q-E-A-G-A-I-K-
   INFORMATION FOR SEQUENCE ID NO 87
      (A) LENGTH:
      (B) TYPE: amino acid
      (D) TOPOLOGY: linear
         -E-K-A-L-S-K-L-H-E-Y-
   INFORMATION FOR SEQUENCE ID NO 88
      (A) LENGTH:
      (B) TYPE: amino acid
      (D) TOPOLOGY: linear
         -E-K-A-L-V-K-L-Q-E-F-
   INFORMATION FOR SEQUENCE ID NO 89
      (A) LENGTH:
      (B) TYPE: amino acid
      (D) TOPOLOGY: linear
         -E-Q-A-L-A-R-L-Q-E-E-
   INFORMATION FOR SEQUENCE ID NO 90
      (A) LENGTH:
      (B) TYPE: amino acid
      (D) TOPOLOGY: linear
         -E-Q-A-L-H-A-L-Q-T-M-
   INFORMATION FOR SEQUENCE ID NO 91
      (A) LENGTH:
      (B) TYPE: amino acid
      (D) TOPOLOGY: linear
         -C-K-D-N-P-G-E-N-A-R-
   INFORMATION FOR SEQUENCE ID NO 92
      (A) LENGTH:
      (B) TYPE: amino acid
      (D) TOPOLOGY: linear
         -S-T-E-T-P-G-K-N-A-C-
   INFORMATION FOR SEQUENCE ID NO 93
      (A) LENGTH:
      (B) TYPE: amino acid
      (D) TOPOLOGY: linear
         -N-I-H-G-S-G-L-N-A-S-
   INFORMATION FOR SEQUENCE ID NO 94
      (A) LENGTH:
      (B) TYPE: amino acid
      (D) TOPOLOGY: linear
         -H-N-R-S-K-I-N-L-Q-D-
   INFORMATION FOR SEQUENCE ID NO 95
      (A) LENGTH:
      (B) TYPE: amino acid
      (D) TOPOLOGY: linear
         -H-N-R-S-A-L-N-I-D-E-

   **GLUCAGON PRECURSOR**
      INFORMATION FOR SEQUENCE ID NO 96
         (A) LENGTH:
         (B) TYPE: amino acid
         (D) TOPOLOGY: linear
            -D-Y-S-K-Y-L-D-S-
      INFORMATION FOR SEQUENCE ID NO 97
         (A) LENGTH:
         (B) TYPE: amino acid
         (D) TOPOLOGY: linear
            -D-S-R-R-A-Q-D-
      INFORMATION FOR SEQUENCE ID NO 98
         (A) LENGTH:
         (B) TYPE: amino acid
         (D) TOPOLOGY: linear
            -D-Y-S-K-Y-L-D-S-R-R-A-Q-D-
      INFORMATION FOR SEQUENCE ID NO 99
         (A) LENGTH:
         (B) TYPE: amino acid
         (D) TOPOLOGY: linear
            -D-V-A-S-L-T-D-Y-L-K-S-K-R-
      INFORMATION FOR SEQUENCE ID NO 100
         (A) LENGTH:
         (B) TYPE: amino acid
         (D) TOPOLOGY: linear
            -D-Y-S-K-Y-M-D-N-R-R-A-K-D-
      INFORMATION FOR SEQUENCE ID NO 101
         (A) LENGTH:
         (B) TYPE: amino acid
         (D) TOPOLOGY: linear
            -D-Y-S-K-Y-L-D-N-R-R-A-K-D-
      INFORMATION FOR SEQUENCE ID NO 102
         (A) LENGTH:
         (B) TYPE: amino acid
         (D) TOPOLOGY: linear
            -D-Y-S-K-Y-L-D-S-R-R-A-Q-Q-
      INFORMATION FOR SEQUENCE ID NO 103
         (A) LENGTH:
         (B) TYPE: amino acid
         (D) TOPOLOGY: linear
            -D-Y-S-K-Y-L-D-S-
      INFORMATION FOR SEQUENCE ID NO 104
         (A) LENGTH:
         (B) TYPE: amino acid
         (D) TOPOLOGY: linear
            -D-S-R-R-A-Q-D-
      INFORMATION FOR SEQUENCE ID NO 105
         (A) LENGTH:
         (B) TYPE: amino acid
         (D) TOPOLOGY: linear
            -D-Y-S-K-Y-L-D-S-R-R-A-Q-D-
      INFORMATION FOR SEQUENCE ID NO 106
         (A) LENGTH :
         (B) TYPE: amino acid
         (D) TOPOLOGY: linear
            -D-V-A-S-L-T-D-Y-L-K-S-K-R-
      INFORMATION FOR SEQUENCE ID NO 107
         (A) LENGTH:
         (B) TYPE: amino acid
         (D) TOPOLOGY: linear
            -D-Y-S-K-Y-M-D-N-R-R-A-K-D-
      INFORMATION FOR SEQUENCE ID NO 108
         (A) LENGTH:
         (B) TYPE: amino acid
         (D) TOPOLOGY: linear
            -D-Y-S-K-Y-L-D-N-R-R-A-K-D-
      INFORMATION FOR SEQUENCE ID NO 109
         (A) LENGTH:
         (B) TYPE : amino acid
         (D) TOPOLOGY: linear
            -D-Y-S-K-Y-L-D-S-R-R-A-Q-Q-
      INFORMATION FOR SEQUENCE ID NO 110
         (A) LENGTH:
         (B) TYPE: amino acid
         (D) TOPOLOGY: linear
            -D-Y-S-K-F-L-D-T-R-R-A-Q-D-
      INFORMATION FOR SEQUENCE ID NO 111
         (A) LENGTH:
         (B) TYPE: amino acid
         (D) TOPOLOGY: linear
            -D-Y-S-K-Y-Q-E-E-R-M-A-Q-D-
      INFORMATION FOR SEQUENCE ID NO 112
         (A) LENGTH:
         (B) TYPE: amino acid
         (D) TOPOLOGY: linear
            -D-Y-S-K-Y-L-E-T-R-R-A-Q-D-
      INFORMATION FOR SEQUENCE ID NO 113
         (A) LENGTH:
         (B) TYPE : amino acid
         (D) TOPOLOGY: linear
            -D-Y-S-K-Y-L-D-N-R-R-T-K-D-
      INFORMATION FOR SEQUENCE ID NO 114
         (A) LENGTH:
         (B) TYPE: amino acid
         (D) TOPOLOGY: linear
            -D-M-S-S-Y-L-E-E-K-A-A-K-E-
      INFORMATION FOR SEQUENCE ID NO 115
         (A) LENGTH:
         (B) TYPE: amino acid
         (D) TOPOLOGY: linear
            -D-F-N-K-A-L-D-I-K-A-A-O-E-
      INFORMATION FOR SEQUENCE ID NO 116
         (A) LENGTH:
         (B) TYPE: amino acid
         (D) TOPOLOGY: linear
            -D-P-L-S-D-P-D-Q-M-
      INFORMATION FOR SEQUENCE ID NO 117
         (A) LENGTH:
         (B) TYPE: amino acid
         (D) TOPOLOGY: linear
            -N-E-D-K-R-H-S-Q-
      INFORMATION FOR SEQUENCE ID NO 118
         (A) LENGTH:
         (B) TYPE: amino acid
         (D) TOPOLOGY linear
            -K-D-E-P-R-E-L-S-N-M-K-R-H-S-E-
      INFORMATION FOR SEQUENCE ID NO 119
         (A) LENGTH:
         (B) TYPE: amino acid
         (D) TOPOLOGY: linear
            -R-E-L-S-N-M-K-R-H-
      INFORMATION FOR SEQUENCE ID NO 120
         (A) LENGTH:
         (B) TYPE: amino acid
         (D) TOPOLOGY: linear
            -E-A-R-E-L-S-T-P-K-X-H-S-E-
      INFORMATION FOR SEQUENCE ID NO 121
         (A) LENGTH:
         (B) TYPE: amino acid
         (D) TOPOLOGY: linear
            -E-D-P-D-Q-I-N-E-D-K-R-H-
      INFORMATION FOR SEQUENCE ID NO 122
         (A) LENGTH:
         (B) TYPE: amino acid
         (D) TOPOLOGY: linear
            -D-D-P-D-Q-M-N-E-D-K-R-H-S-Q-
      INFORMATION FOR SEQUENCE ID NO 123
         (A) LENGTH:
         (B) TYPE: amino acid
         (D) TOPOLOGY: linear
            -G-D-P-D-Q-I-N-E-D-K-R-H-S-Q-
      INFORMATION FOR SEQUENCE ID NO 124
         (A) LENGTH:
         (B) TYPE: amino acid
         (D) TOPOLOGY: linear
            -D-E-S-R-Q-L-N-E-V-K-R-H-S-Q-
      INFORMATION FOR SEQUENCE ID NO 125
         (A) LENGTH:
         (8) TYPE: amino acid
         (D) TOPOLOGY: linear
            -M-N-T-K-R-N-R-N-N-
      INFORMATION FOR SEQUENCE ID NO 126
         (A) LENGTH:
         (B) TYPE: amino acid
         (D) TOPOLOGY: linear
            -K-R-H-D-E-F-E-R-H-
      INFORMATION FOR SEQUENCE ID NO 127
         (A) LENGTH:
         (B) TYPE: amino acid
         (D) TOPOLOGY: linear
            -M-N-T-K-R-N-R-N-R-N-N-K-R-H-D-E-F-E-R-H-
      INFORMATION FOR SEQUENCE ID NO 128
         (A) LENGTH:
         (B) TYPE: amino acid
         (D) TOPOLOGY: linear
            -N-K-R-S-G-V-A-E-K-R-
      INFORMATION FOR SEQUENCE ID NO 129
         (A) LENGTH:
         (B) TYPE: amino acid
         (D) TOPOLOGY: linear
            -V-K-R-N-R-N-N-I-A-K-R-
      INFORMATION FOR SEQUENCE ID NO 130
         (A) LENGTH:
         (B) TYPE: amino acid
         (D) TOPOLOGY: linear
            -K-N-T-K-R-N-R-N-E-
      INFORMATION FOR SEQUENCE ID NO 131
         (A) LENGTH:
         (B) TYPE: amino acid
         (D) TOPOLOGY: linear
            -T-K-R-N-G-O-O-G-O-E-D-K-
      INFORMATION FOR SEQUENCE ID NO 132
         (A) LENGTH:
         (B) TYPE: amino acid
         (D) TOPOLOGY: linear
            -Q-E-D-K-E-N-D-K-F-
      INFORMATION FOR SEQUENCE ID NO 133
         (A) LENGTH:
         (B) TYPE: amino acid
         (D) TOPOLOGY: linear
            -K-R-H-S-E-F-E-R-H-A-E-
      INFORMATION FOR SEQUENCE ID NO 134
         (A) LENGTH:
         (B) TYPE: amino acid
         (D) TOPOLOGY: linear
            -Q-D-T-E-E-K-S-R-
      INFORMATION FOR SEQUENCE ID NO 135
         (A) LENGTH:
         (B) TYPE: amino acid
         (D) TOPOLOGY: linear
            -Q-D-T-E-E-K-P-R-
      INFORMATION FOR SEQUENCE ID NO 136
         (A) LENGTH:
         (B) TYPE: amino acid
         (D) TOPOLOGY: linear
            -Q-D-T-E-E-N-A-R-
      INFORMATION FOR SEQUENCE ID NO 137
         (A) LENGTH:
         (B) TYPE: amino acid
         (D) TOPOLOGY: linear
            -D-Q-D-P-D-R-N-S-M-
      INFORMATION FOR SEQUENCE ID NO 138
         (A) LENGTH:
         (B) TYPE: amino acid
         (D) TOPOLOGY: linear
            -K-G-R-G-R-R-D-F-P-E-E-
      INFORMATION FOR SEQUENCE ID NO 139
         (A) LENGTH:
         (B) TYPE: amino acid
         (D) TOPOLOGY: linear
            -D-R-L-K-A-Q-V-R-R-E-
      INFORMATION FOR SEQUENCE ID NO 140
         (A) LENGTH:
         (B) TYPE: amino acid
         (D) TOPOLOGY: linear
            -A-K-L-K-S-G-K-V-
      INFORMATION FOR SEQUENCE ID NO 141
         (A) LENGTH:
         (B) TYPE: amino acid
         (D) TOPOLOGY: linear
            -K-S-G-Q-P-K-P-E-
      INFORMATION FOR SEQUENCE ID NO 142
         (A) LENGTH:
         (B) TYPE: amino acid
         (D) TOPOLOGY: linear
            -K-O-G-Q-D-R-R-E-
**GASTRIN PRECURSOR**
   INFORMATION FOR SEQUENCE ID NO 143
      (A) LENGTH:
      (B) TYPE: amino acid
      (D) TOPOLOGY: linear
         -A-D-P-S-K-K-Q-
   INFORMATION FOR SEQUENCE ID NO 144
      (A) LENGTH:
      (B) TYPE: amino acid
      (D) TOPOLOGY: linear
         -R-T-D-L-S-K-K-Q-
   INFORMATION FOR SEQUENCE ID NO 145
      (A) LENGTH:
      (B) TYPE: amino acid
      (D) TOPOLOGY: linear
         -A-D-L-S-K-K-Q-
   INFORMATION FOR SEQUENCE ID NO 146
      (A) LENGTH:
      (B) TYPE: amino acid
      (D) TOPOLOGY: linear
         -D-L-S-K-K-Q-R-
   INFORMATION FOR SEQUENCE ID NO 147
      (A) LENGTH:
      (B) TYPE: amino acid
      (D) TOPOLOGY: linear
         -W-L-E-E-E-E-E-A-
   INFORMATION FOR SEQUENCE ID NO 148
      (A) LENGTH:
      (B) TYPE: amino acid
      (D) TOPOLOGY: linear
         -W-M-E-E-E-E-A-
   INFORMATION FOR SEQUENCE ID NO 149
      (A) LENGTH:
      (B) TYPE: amino acid
      (D) TOPOLOGY: linear
         -W-V-E-E-E-E-A-
   INFORMATION FOR SEQUENCE ID NO 150
      (A) LENGTH:
      (B) TYPE: amino acid
      (D) TOPOLOGY: linear
         -W-A-E-E-E-E-A-
   INFORMATION FOR SEQUENCE ID NO 151
      (A) LENGTH:
      (B) TYPE: amino acid
      (D) TOPOLOGY: linear
         -P-M-E-E-E-E-E-A-
   INFORMATION FOR SEQUENCE ID NO 152
      (A) LENGTH:
      (B) TYPE: amino acid
      (D) TOPOLOGY: linear
         -D-F-G-R-R-S-A-E-D-E-N-
   INFORMATION FOR SEQUENCE ID NO 153
      (A) LENGTH:
      (B) TYPE: amino acid
      (D) TOPOLOGY: linear
         -M-D-F-G-R-R-S-
   INFORMATION FOR SEQUENCE ID NO 154
      (A) LENGTH:
      (B) TYPE: amino acid
      (D) TOPOLOGY: linear
         -R-R-S-A-E-D-E-
   INFORMATION FOR SEQUENCE ID NO 155
      (A) LENGTH:
      (B) TYPE: amino acid
      (D) TOPOLOGY: linear
         -R-R-S-A-E-D-G-D-Q-H-P-
   INFORMATION FOR SEQUENCE ID NO 156
      (A) LENGTH:
      (B) TYPE: amino acid
      (D) TOPOLOGY: linear
         -R-D-L-E-L-P-W-L-E-
   INFORMATION FOR SEQUENCE ID NO 157
      (A) LENGTH:
      (B) TYPE: amino acid
      (D) TOPOLOGY: linear
         -R-G-K-E-P-H-E-L-D-R-
   INFORMATION FOR SEQUENCE ID NO 158
      (A) LENGTH:
      (8) TYPE: ammo acid
      (D) TOPOLOGY: linear
         -R-G-Q-E-P-L-R-
   INFORMATION FOR SEQUENCE ID NO 159
      (A) LENGTH:
      (B) TYPE: amino acid
      (D) TOPOLOGY: linear
         -E-P-H-W-L-N-R-
   INFORMATION FOR SEQUENCE ID NO 160
      (A) LENGTH:
      (B) TYPE: amino acid
      (D) TOPOLOGY: linear
         -K-P-R-S-Q-Q-P-D-
   INFORMATION FOR SEQUENCE ID NO 161
      (A) LENGTH:
      (B) TYPE: amino acid
      (D) TOPOLOGY: linear
         -K-P-G-F-Q-L-Q-D-
   INFORMATION FOR SEQUENCE ID NO 162
      (A) LENGTH:
      (B) TYPE: amino acid
      (D) TOPOLOGY: linear
         -K-P-H-S-H-L-Q-D-
   INFORMATION FOR SEQUENCE ID NO 163
      (A) LENGTH:
      (B) TYPE: amino acid
      (D) TOPOLOGY: linear
         -K-P-R-S-Q-L-Q-D-
**GONADOLIBERIN PRECURSOR**
   INFORMATION FOR SEQUENCE ID NO 164
      (A) LENGTH:
      (B) TYPE: amino acid
      (D) TOPOLOGY: linear
         -Q-H-W-S-Y-G-L-R-P-G-G-K-
   INFORMATION FOR SEQUENCE ID NO 165
      (A) LENGTH:
      (B) TYPE: amino acid
      (D) TOPOLOGY: linear
         -G-L-Q-P-G-G-K-R-D-
   INFORMATION FOR SEQUENCE ID NO 166
      (A) LENGTH:
      (B) TYPE: amino acid
      (D) TOPOLOGY: linear
         -H-G-W-L-P-G-G-K-R-D-
   INFORMATION FOR SEQUENCE ID NO 167
      (A) LENGTH:
      (B) TYPE: amino acid
      (D) TOPOLOGY: linear
         -G-W-L-P-G-G-K-R-D-
   INFORMATION FOR SEQUENCE ID NO 168
      (A) LENGTH:
      (B) TYPE: amino acid
      (D) TOPOLOGY: linear
         -H-G-W-Y-P-G-G-K-R-D-
   INFORMATION FOR SEQUENCE ID NO 169
      (A) LENGTH:
      (B) TYPE: amino acid
      (D) TOPOLOGY: linear
         -Q-H-Y-S-L-E-W-K-P-G-
   INFORMATION FOR SEQUENCE ID NO 170
      (A) LENGTH:
      (B) TYPE: amino acid
      (D) TOPOLOGY: linear
         -R-P-G-G-K-R-D-A-E-
   INFORMATION FOR SEQUENCE lD NO 171
      (A) LENGTH:
      (B) TYPE: amino acid
      (D) TOPOLOGY: linear
         -R-P-G-G-K-R-N-T-E-H-
   INFORMATION FOR SEQUENCE ID NO 172
      (A) LENGTH:
      (B) TYPE: amino acid
      (D) TOPOLOGY: linear
         -Q-P-G-G-K-R-D-A-E-
**PLASMINOGEN ACTIVATOR INHIBITOR 1**
   INFORMATION FOR SEQUENCE ID NO 173
      (A) LENGTH:
      (B) TYPE: amino acid
      (D) TOPOLOGY: linear
         -Q-A-S-K-D-R-
   INFORMATION FOR SEQUENCE ID NO 174
      (A) LENGTH:
      (B) TYPE: amino acid
      (D) TOPOLOGY: linear
         -K-I-D-D-K-G-
   INFORMATION FOR SEQUENCE ID NO 175
      (A) LENGTH:
      (B) TYPE: amino acid
      (D) TOPOLOGY: linear
         -W-N-K-D-E-
   INFORMATION FOR SEQUENCE ID NO 176
      (A) LENGTH:
      (B) TYPE: amino acid
      (D) TOPOLOGY: linear
         -E-V-E-R-A-R-
   INFORMATION FOR SEQUENCE ID NO 177
      (A) LENGTH:
      (B) TYPE: amino acid
      (D) TOPOLOGY: linear
         -H-R-R-L-F-H-K-S-D-
   INFORMATION FOR SEQUENCE ID NO 178
      (A) LENGTH:
      (B) TYPE: amino acid
      (D) TOPOLOGY: linear
         -K-T-P-F-P-D-S-
   INFORMATION FOR SEQUENCE ID NO 179
      (A) LENGTH
      (B) TYPE: amino acid
      (D) TOPOLOGY: linear
         -Y-E-K-E-V-P-
   INFORMATION FOR SEQUENCE ID NO 180
      (A) LENGTH:
      (B) TYPE: amino acid
      (D) TOPOLOGY: linear
         -E-V-D-L-R-K-P-
   INFORMATION FOR SEQUENCE ID NO 181
      (A) LENGTH:
      (B) TYPE: amino acid
      (D) TOPOLOGY: linear
         -K-V-K-I-E-V-N-E-
   INFORMATION FOR SEQUENCE ID NO 182
      (A) LENGTH:
      (B) TYPE: amino acid
      (D) TOPOLOGY: linear
         -A-R-M-A-P-E-E-
   INFORMATION FOR SEQUENCE ID NO 183
      (A) LENGTH:
      (B) TYPE: amino acid
      (D) TOPOLOGY: linear
         -R-A-S-K-D-R-
   INFORMATION FOR SEQUENCE ID NO 184
      (A) LENGTH:
      (B) TYPE: amino acid
      (D) TOPOLOGY: linear
         -K-I-E-E-K-G-
   INFORMATION FOR SEQUENCE ID NO 185
      (A) LENGTH:
      (B) TYPE: amino acid
      (D) TOPOLOGY: linear
         -K-V-N-E-K-G-
   INFORMATION FOR SEQUENCE ID NO 186
      (A) LENGTH:
      (B) TYPE: amino acid
      (D) TOPOLOGY: linear
         -N-I-S-E-R-G-
   INFORMATION FOR SEQUENCE ID NO 187
      (A) LENGTH:
      (B) TYPE: amino acid
      (D) TOPOLOGY: linear
         -W-N-K-N-E-
   INFORMATION FOR SEQUENCE ID NO 188
      (A) LENGTH:
      (B) TYPE: amino acid
      (D) TOPOLOGY: linear
         -K-M-P-F-P-E-S-
   INFORMATION FOR SEQUENCE ID NO 189
      (A) LENGTH:
      (B) TYPE: amino acid
      (D) TOPOLOGY: linear
         -K-T-P-F-L-E-A-
   INFORMATION FOR SEQUENCE ID NO 190
      (A) LENGTH:
      (B) TYPE: amino acid
      (D) TOPOLOGY: linear
         -H-H-R-L-F-H-K-S-D-
   INFORMATION FOR SEQUENCE ID NO 191
      (A) LENGTH:
      (B) TYPE: amino acid
      (D) TOPOLOGY: linear
         -H-Q-R-L-F-H-K-S-D-
   INFORMATION FOR SEQUENCE ID NO 192
      (A) LENGTH:
      (B) TYPE: amino acid
      (D) TOPOLOGY: linear
         -E-V-D-L-R-G-P-L-E-K-
   INFORMATION FOR SEQUENCE ID NO 193
      (A) LENGTH:
      (B) TYPE: amino acid
      (D) TOPOLOGY: linear
         -E-I-D-L-R-R-P-L-E-
   INFORMATION FOR SEQUENCE ID NO 194
      (A) LENGTH:
      (B) TYPE: amino acid
      (D) TOPOLOGY: linear
         -A-R-M-A-P-T-E-M-
**PLASMINOGEN ACTIVATOR INHIBITOR 2**
   INFORMATION FOR SEQUENCE ID NO 195
      (A) LENGTH:
      (B) TYPE: amino acid
      (D) TOPOLOGY: linear
         -K-W-K-T-P-F-E-K-
   INFORMATION FOR SEQUENCE ID NO 196
      (A) LENGTH:
      (B) TYPE: amino acid
      (D) TOPOLOGY: linear
         -R-E-K-L-N-I-G-Y-I-E-D-L-K-
   INFORMATION FOR SEQUENCE ID NO 197
      (A) LENGTH:
      (B) TYPE: amino acid
      (D) TOPOLOGY: linear
         -H-A-K-L-N-I-G-Y-I-K-D-L-K-
   INFORMATION FOR SEQUENCE ID NO 198
      (A) LENGTH:
      (B) TYPE: amino acid
      (D) TOPOLOGY: linear
         -K-D-K-M-A-E-D-E-V-E-
   INFORMATION FOR SEQUENCE ID NO 199
      (A) LENGTH:
      (B) TYPE: amino acid
      (D) TOPOLOGY: linear
         -K-D-T-L-D-E-D-D-
   INFORMATION FOR SEQUENCE ID NO 200
      (A) LENGTH:
      (B) TYPE: amino acid
      (D) TOPOLOGY: linear
         -K-L-E-E-H-Y-E-L-R-
   INFORMATION FOR SEQUENCE ID NO 201
      (A) LENGTH:
      (B) TYPE: amino acid
      (D) TOPOLOGY: linear
         -K-L-A-O-S-Y-E-L-K-
   INFORMATION FOR SEQUENCE lD NO 202
      (A) LENGTH:
      (B) TYPE: amino acid
      (D) TOPOLOGY: linear
         -E-R-N-D-L-F-L-S-E-
   INFORMATION FOR SEQUENCE ID NO 203
      (A) LENGTH:
      (B) TYPE: amino acid
      (D) TOPOLOGY: linear
         -D-V-N-E-E-G-T-E-
   INFORMATION FOR SEQUENCE ID NO 204
      (A) LENGTH:
      (B) TYPE: amino acid
      (D) TOPOLOGY: linear
         -D-V-T-E-E-G-T-V-
   INFORMATION FOR SEQUENCE ID NO 205
      (A) LENGTH:
      (B) TYPE: amino acid
      (D) TOPOLOGY: linear
         -R-G-S-T-E-D-E-Q-
   INFORMATION FOR SEQUENCE ID NO 206
      (A) LENGTH:
      (B) TYPE: amino acid
      (D) TOPOLOGY: linear
         -G-G-N-T-E-Q-Q-
   INFORMATION FOR SEQUENCE ID NO 207
      (A) LENGTH:
      (B) TYPE: amino acid
      (D) TOPOLOGY: linear
         -E-K-S-A-S-F-R-E-E-
   INFORMATION FOR SEQUENCE ID NO 208
      (A) LENGTH:
      (B) TYPE: amino acid
      (D) TOPOLOGY: linear
         -E-K-S-A-R-F-K-E-E-
   INFORMATION FOR SEQUENCE ID NO 209
      (A) LENGTH:
      (B) TYPE: amino acid
      (D) TOPOLOGY: linear
         -E-E-A-R-K-K-
   INFORMATION FOR SEQUENCE lD NO 210
      (A) LENGTH:
      (B) TYPE: amino acid
      (D) TOPOLOGY: linear
         -E-E-A-R-E-K-
   INFORMATION FOR SEQUENCE ID NO 211
      (A) LENGTH:
      (B) TYPE: amino acid
      (D) TOPOLOGY: linear
         -K-T-Q-T-K-G-K-
   INFORMATION FOR SEQUENCE ID NO 212
      (A) LENGTH:
      (B) TYPE: amino acid
      (D) TOPOLOGY: linear
         -K-T-Q-T-K-G-E-
   INFORMATION FOR SEQUENCE ID NO 213
      (A) LENGTH:
      (B) TYPE: amino acid
      (D) TOPOLOGY: linear
         -E-G-S-V-D-G-D-T-R-
   INFORMATION FOR SEQUENCE ID NO 214
      (A) LENGTH:
      (B) TYPE: amino acid
      (D) TOPOLOGY: linear
         -E-G-S-V-D-E-D-T-K-
**ALZHEIMER AMYLOID A4**
   INFORMATION FOR SEQUENCE ID NO 215
      (A) LENGTH:
      (B) TYPE: amino acid
      (D) TOPOLOGY: linear
         -D-S-A-D-A-E-E-D-D-
   INFORMATION FOR SEQUENCE ID NO 216
      (A) LENGTH:
      (B) TYPE: amino acid
      (D) TOPOLOGY: linear
         -E-E-E-E-V-A-E-V-E-
   INFORMATION FOR SEQUENCE ID NO 217
      (A) LENGTH:
      (B) TYPE: amino acid
      (D) TOPOLOGY: linear
         -E-E-E-E-A-D-D-D-
   INFORMATION FOR SEQUENCE ID NO 218
      (A) LENGTH:
      (B) TYPE: amino acid
      (D) TOPOLOGY: linear
         A-D-D-D-E-D-D-E-D-G-
   INFORMATION FOR SEQUENCE ID NO 219
      (A) LENGTH:
      (B) TYPE: amino acid
      (D) TOPOLOGY: linear
         -D-E-D-G-D-E-V-E-
   INFORMATION FOR SEQUENCE ID NO 220
      (A) LENGTH:
      (B) TYPE: amino acid
      (D) TOPOLOGY: linear
         -E-E-A-E-E-P-Y-E-E-
   INFORMATION FOR SEQUENCE ID NO 221
      (A) LENGTH:
      (B) TYPE: amino acid
      (D) TOPOLOGY: linear
         -A-D-V-E-E-E-E-A-D-D-D-E-D-V-E-
   INFORMATION FOR SEQUENCE ID NO 222
      (A) LENGTH:
      (B) TYPE: amino acid
      (D) TOPOLOGY: linear
         -K-A-K-E-R-L-E-
   INFORMATION FOR SEQUENCE ID NO 223
      (A) LENGTH:
      (B) TYPE: amino acid
      (D) TOPOLOGY: linear
         -E-A-K-H-R-E-R-
   INFORMATION FOR SEQUENCE ID NO 224
      (A) LENGTH:
      (B) TYPE: amino acid
      (D) TOPOLOGY: linear
         -R-E-W-E-E-A-E-R-
   INFORMATION FOR SEQUENCE ID NO 225
      (A) LENGTH:
      (B) TYPE: amino acid
      (D) TOPOLOGY: linear
         -P-A-E-Q-K-D-R-
**APOLIPOPROTEIN E**
   INFORMATION FOR SEQUENCE ID NO 226
      (A) LENGTH:
      (B) TYPE: amino acid
      (D) TOPOLOGY: linear
         -E-P-E-T-E-F-D-V-R-
   INFORMATION FOR SEQUENCE ID NO 227
      (A) LENGTH:
      (B)TYPE: amino acid
      (D) TOPOLOGY: linear
         -E-G-E-P-E-V-T-D-
   INFORMATION FOR SEQUENCE ID NO 228
      (A) LENGTH:
      (B) TYPE: amino acid
      (D) TOPOLOGY: linear
         -E-T-E-P-E-P-E-L-R-
   INFORMATION FOR SEQUENCE ID NO 229
      (A) LENGTH:
      (B) TYPE: amino acid
      (D) TOPOLOGY: linear
         -E-D-V-C-G-R-
   INFORMATION FOR SEQUENCE ID NO 230
      (A) LENGTH:
      (B) TYPE: amino acid
      (D) TOPOLOGY: linear
         -E-D-V-R-G-R-
   INFORMATION FOR SEQUENCE ID NO 231
      (A) LENGTH:
      (B) TYPE: amino acid
      (D) TOPOLOGY: linear
         -E-D-L-R-N-R-
   INFORMATION FOR SEQUENCE ID NO 232
      (A) LENGTH:
      (B) TYPE: amino acid
      (D) TOPOLOGY: linear
         -H-L-R-K-L-R-K-R-
   INFORMATION FOR SEQUENCE ID NO 233
      (A) LENGTH:
      (B) TYPE: amino acid
      (D) TOPOLOGY: linear
         -H-P-R-K-M-K-R-R-
   INFORMATION FOR SEQUENCE ID NO 234
      (A) LENGTH:
      (B) TYPE: amino acid
      (D) TOPOLOGY: linear
         -H-L-R-K-M-R-K-R-
   INFORMATION FOR SEQUENCE ID NO 235
      (A) LENGTH:
      (B) TYPE: amino acid
      (D) TOPOLOGY: linear
         -R-D-R-L-D-E-V-K-E-
   INFORMATION FOR SEQUENCE ID NO 236
      (A) LENGTH:
      (B) TYPE: amino acid
      (D) TOPOLOGY: linear
         -R-A-R-M-E-E-
   INFORMATION FOR SEQUENCE ID NO 237
      (A) LENGTH:
      (B) TYPE: amino acid
      (D) TOPOLOGY: linear
         -R-G-R-M-E-E-
   INFORMATION FOR SEQUENCE ID NO 238
      (A) LENGTH:
      (B) TYPE: amino acid
      (D) TOPOLOGY: linear
         -R-G-R-L-E-E-
**HERPES VIRUS 1 (HSV1) GLYCOPROTEIN B**
   INFORMATION FOR SEQUENCE ID NO 239
      (A) LENGTH:
      (B) TYPE: amino acid
      (D) TOPOLOGY: linear
         -D-Y-T-E-V-O-R-R-N-O-L-H-D-
   INFORMATION FOR SEQUENCE lD NO 240
      (A) LENGTH:
      (B) TYPE: ammo acid
      (D) TOPOLOGY: linear
         -K-E-L-K-N-P-T-N-P-D-
   INFORMATION FOR SEQUENCE ID NO 242
      (A) LENGTH:
      (B) TYPE: amino acid
      (D) TOPOLOGY: linear
         -E-G-E-E-G-G-D-F-D-E-A-K-
   INFORMATION FOR SEQUENCE ID NO 242
      (A) LENGTH:
      (B) TYPE: amino acid
      (D) TOPOLOGY: linear
         -D-E-A-K-L-A-E-A-R-E-M-I-R-
   INFORMATION FOR SEQUENCE ID NO 243
      (A) LENGTH:
      (B) TYPE: amino acid
      (D) TOPOLOGY: linear
         -D-M-V-M-R-K-R-R-N-
   INFORMATION FOR SEQUENCE ID NO 244
      (A) LENGTH:
      (B) TYPE: amino acid
      (D) TOPOLOGY: linear
         -R-K-R-R-N-T-N-Y-T-Q-V-P-N-K-D-A-D-E-D-D-L-
**HERPES VIRUS 2 (HSV 23, 2H) GLYCOPROTEIN B**
   INFORMATION FOR SEQUENCE ID NO 245
      (A) LENGTH:
      (B) TYPE: amino acid
      (D) TOPOLOGY: linear
         -K-A-R-K-R-K-T-K-K-
   INFORMATION FOR SEQUENCE ID NO 246
      (A) LENGTH:
      (B) TYPE: amino acid
      (D) TOPOLOGY: linear
         -K-K-P-P-K-R-P-E-
   INFORMATION FOR SEQUENCE ID NO 247
      (A) LENGTH:
      (B) TYPE: amino acid
      (D) TOPOLOGY: linear
         -R-D-A-R-E-A-I-D-R-
   INFORMATION FOR SEQUENCE ID NO 248
      (A) LENGTH:
      (B) TYPE: amino acid
      (D) TOPOLOGY: linear
         -R-E-Y-M-R-E-Q-D-R-K-
   INFORMATION FOR SEQUENCE ID NO 249
      (A) LENGTH:
      (B) TYPE: amino acid
      (D) TOPOLOGY: linear
         -R-K-R-N-K-A-R-
   INFORMATION FOR SEQUENCE ID NO 250
      (A) LENGTH:
      (B) TYPE: amino acid
      (D) TOPOLOGY: linear
         -E-D-E-A-G-D-E-D-E-L-
   INFORMATION FOR SEQUENCE ID NO 251
      (A) LENGTH:
      (B) TYPE: amino acid
      (D) TOPOLOGY: linear
         -R-K-R-N-K-A-R-Y-S-P-L-H-N-E-D-E-A-G-D-E-D-E-L-
**TREPONEMA PALLIDUM MEMBRANE PROTEIN TMPA**
   INFORMATION FOR SEQUENCE ID NO 252
      (A) LENGTH:
      (B) TYPE: amino acid
      (D) TOPOLOGY: linear
         -K-E-E-A-E-K-K-A-A-E-Q-R-
   INFORMATION FOR SEQUENCE ID NO 253
      (A) LENGTH:
      (B) TYPE: amino acid
      (D) TOPOLOGY: linear
         -H-A-D-R-R-L-M-E-
   INFORMATION FOR SEQUENCE ID NO 254
      (A) LENGTH:
      (B) TYPE: amino acid
      (D) TOPOLOGY: linear
         -D-V-E-R-Q-S-T-D-A-K-
   INFORMATION FOR SEQUENCE ID NO 255
      (A) LENGTH:
      (B) TYPE: amino acid
      (D) TOPOLOGY: linear
         -K-I-E-G-D-L-K-K-
   INFORMATION FOR SEQUENCE ID NO 256
      (A) LENGTH:
      (B) TYPE: amino acid
      (D) TOPOLOGY: linear
         -E-L-R-P-Q-L-E-E-A-D-
   INFORMATION FOR SEQUENCE ID NO 257
      (A) LENGTH:
      (B) TYPE: amino acid
      (D) TOPOLOGY: linear
         -D-S-Q-Y-Q-E-A-R-E-A-E-E-
   INFORMATION FOR SEQUENCE ID NO 258
      (A) LENGTH:
      (B) TYPE: amino acid
      (D) TOPOLOGY: linear
         -R-L-K-K-T-E-A-E-K-
   INFORMATION FOR SEQUENCE ID NO 259
      (A) LENGTH:
      (B) TYPE: amino acid
      (D) TOPOLOGY: linear
         -A-K-T-K-Q-K-A-
   INFORMATION FOR SEQUENCE ID NO 260
      (A) LENGTH:
      (B) TYPE: amino acid
      (D) TOPOLOGY: linear
         -D-L-A-R-S-A-D-K-S-
   INFORMATION FOR SEQUENCE ID NO 261
      (A) LENGTH:
      (B) TYPE: amino acid
      (D) TOPOLOGY: linear
         -K-E-P-I-E-V-E-P-L- P-N-D-R-
   INFORMATION FOR SEQUENCE ID NO 262
      (A) LENGTH:
      (B) TYPE: amino acid
      (D) TOPOLOGY: linear
         -R-V-Q-S-R-G-V-E-D-G-G-R-S-P-K-
**ISLET AMYLOID POLYPEPTIDE**
   INFORMATION FOR SEQUENCE ID NO 263
      (A) LENGTH:
      (B) TYPE: amino acid
      (D) TOPOLOGY: linear
         -E-S-H-Q-V-E-K-R-K-
   INFORMATION FOR SEQUENCE ID NO 264
      (A) LENGTH:
      (B) TYPE: amino acid
      (D) TOPOLOGY: linear
         -N-P-O-M-D-K-R-K-
   INFORMATION FOR SEQUENCE ID NO 265
      (A) LENGTH:
      (B) TYPE: amino acid
      (D) TOPOLOGY: linear
         -D-H-R-V-D-K-R-K-
   INFORMATION FOR SEQUENCE ID NO 266
      (A) LENGTH:
      (B) TYPE: amino acid
      (D) TOPOLOGY: linear
         -K-R-N-A-V-E-V-L-K-R-E-
   INFORMATION FOR SEQUENCE ID NO 267
      (A) LENGTH:
      (B) TYPE: amino acid
      (D) TOPOLOGY: linear
         -K-R-N-V-A-E-D-P-N-R-E-
   INFORMATION FOR SEQUENCE ID NO 268
      (A) LENGTH:
      (B) TYPE: amino acid
      (D) TOPOLOGY: linear
         -R-R-N-A-E-V-V-D-V-E-
**COLLAGENASE (FIBROBLAST MMP1)**
   INFORMATION FOR SEQUENCE ID NO 269
      (A) LENGTH:
      (B) TYPE: amino acid
      (D) TOPOLOGY: linear
         -L-K-N-D-G-R-Q-V-E-K-R-R-N-
   INFORMATION FOR SEQUENCE ID NO 270
      (A) LENGTH:
      (B) TYPE: amino acid
      (D) TOPOLOGY: linear
         -K-N-D-G-R-Q-V-E-K-R-R-
   INFORMATION FOR SEQUENCE ID NO 271
      (A) LENGTH:
      (B) TYPE: amino acid
      (D) TOPOLOGY: linear
         -K-D-D-W-R-K-I-P-K-Q-R-
   INFORMATION FOR SEQUENCE ID NO 272
      (A) LENGTH:
      (B) TYPE : ami no acid
      (D) TOPOLOGY: linear
         -K-K-V-E-R-Q-R-
   INFORMATION FOR SEQUENCE ID NO 273
      (A) LENGTH:
      (B) TYPE: amino acid
      (D) TOPOLOGY: linear
         -D-G-V-P-V-E-K-K-R-
   INFORMATION FOR SEQUENCE ID NO 274
      (A) LENGTH:
      (B) TYPE: amino acid
      (D) TOPOLOGY: linear
         -R-G-D-H-R-D-N-
   INFORMATION FOR SEQUENCE ID NO 275
      (A) LENGTH:
      (B) TYPE: amino acid
      (D) TOPOLOGY: linear
         -D-E-H-E-R-W-
   INFORMATION FOR SEQUENCE ID NO 276
      (A) LENGTH:
      (B) TYPE: amino acid
      (D) TOPOLOGY: linear
         -D-E-D-D-R-W-T-K-D-
   INFORMATION FOR SEQUENCE ID NO 277
      (A) LENGTH:
      (B) TYPE: amino acid
      (D) TOPOLOGY: linear
         -E-F-A-D-R-D-E-V-R-
   INFORMATION FOR SEQUENCE ID NO 278
      (A) LENGTH:
      (B) TYPE: amino acid
      (D) TOPOLOGY: linear
         -E-V-A-H-R-D-E-
   INFORMATION FOR SEQUENCE ID NO 279
      (A) LENGTH:
      (B) TYPE: amino acid
      (D) TOPOLOGY: linear
         -E-V-A-D-R-D-E-V-R-
   INFORMATION FOR SEQUENCE ID NO 280
      (A) LENGTH:
      (B) TYPE. amino acid
      (D) TOPOLOGY linear
         -R-Y-D-E-Y-K-R-
**SCHISTOSOMA ELASTASE PRECURSOR**
   INFORMATION FOR SEQUENCE ID NO 281
      (A) LENGTH:
      (B) TYPE: amino acid
      (D) TOPOLOGY: linear
         -R-S-G-E-P-
   INFORMATION FOR SEQUENCE ID NO 282
      (A) LENGTH:
      (B) TYPE: amino acid
      (D) TOPOLOGY: linear
         -R-N-G-D-Q-Q-G-I-H-H-
   INFORMATION FOR SEQUENCE ID NO 283
      (A) LENGTH:
      (B) TYPE: amino acid
      (D) TOPOLOGY: linear
         -A-R-Q-R-R-P-
   INFORMATION FOR SEQUENCE ID NO 284
      (A) LENGTH:
      (B) TYPE: amino acid
      (D) TOPOLOGY: linear
         -R-D-D-N-D-R-D-P-S-A-K-
**SCHISTOSOMIN**
   INFORMATION FOR SEQUENCE ID NO 285
      (A) LENGTH:
      (B) TYPE: amino acid
      (D) TOPOLOGY: linear
         -K-V-S-K-R-P-D-Y-D-
   INFORMATION FOR SEQUENCE ID NO 286
      (A) LENGTH:
      (B) TYPE: amino acid
      (D) TOPOLOGY: linear
         -E-A-F-E-C-F-E-S-D-P-N-A-K-
   INFORMATION FOR SEQUENCE ID NO 287
      (A) LENGTH:
      (B) TYPE: amino acid
      (D) TOPOLOGY: linear
         -K-C-R-K-K-Y-E-F-C-R-
**APOLIPOPROTEIN(a) HUMAN**
   INFORMATION FOR SEQUENCE ID NO 288
      (A) LENGTH:
      (B) TYPE: amino acid
      (D) TOPOLOGY: linear
         -H-R-H-Q-R-T-P-E-N-Y-P-N-D-
   INFORMATION FOR SEQUENCE ID NO 289
      (A) LENGTH:
      (B) TYPE: amino acid
      (D) TOPOLOGY: linear
         -D-P-S-I-R-W-E-Y-C-
**APOLIPOPROTEIN(a) RHESUS**
   INFORMATION FOR SEQUENCE ID NO 290
      (A) LENGTH:
      (B) TYPE: amino acid
      (D) TOPOLOGY: linear
         -H-O-H-K-R-T-P-E-N-H-P-N-D-D-
   INFORMATION FOR SEQUENCE ID NO 291
      (A) LENGTH:
      (B) TYPE: amino acid
      (D) TOPOLOGY: linear
         -M-D-P-S-V-R-R-E-Y-C-
   INFORMATION FOR SEQUENCE ID NO 292
      (A) LENGTH:
      (B) TYPE: amino acid
      (D) TOPOLOGY: linear
         -R-T-P-E-N-Y-P-N-G-
   INFORMATION FOR SEQUENCE ID NO 293
      (A) LENGTH:
      (B) TYPE: amino acid
      (D) TOPOLOGY: linear
         -L-E-A-P-S-K-Q-A-
   INFORMATION FOR SEQUENCE ID NO 294
      (A) LENGTH:
      (B) TYPE: amino acid
      (D) TOPOLOGY: linear
         -L-E-A-F-L-Q-E-P-T-E-E-
   INFORMATION FOR SEQUENCE ID NO 295
      (A) LENGTH:
      (B) TYPE: amino acid
      (D) TOPOLOGY: linear
         -G-H-L-A-G-G-T-D-R-
**HEPATITIS DELTA ANTIGEN**
   INFORMATION FOR SEQUENCE ID NO 296
      (A) LENGTH:
      (B) TYPE: amino acid
      (D) TOPOLOGY: linear
         -E-R-R-K-D-R-G-G-R-E-D-
   INFORMATION FOR SEQUENCE ID NO 297
      (A) LENGTH:
      (B) TYPE: amino acid
      (D) TOPOLOGY: linear
         -E-S-R-K-N-R-G-G-R-E-E-
   INFORMATION FOR SEQUENCE ID NO 298
      (A) LENGTH:
      (B) TYPE: amino acid
      (D) TOPOLOGY: linear
         -E-S-K-G.K-R-A-G-R-E-Q-
**REV PROTEIN HIV, SIV, VILV, OMVVS**
   INFORMATION FOR SEQUENCE ID NO 299
      (A) LENGTH:
      (8) TYPE: amino acid
      (D) TOPOLOGY: linear
         -K-E-G-F-R-N-K-V-P-C-L-Q-E-
   INFORMATION FOR SEQUENCE ID NO 300
      (A) LENGTH:
      (B) TYPE: amino acid
      (D) TOPOLOGY: linear
         -G-E-E-E-L-R-R-R-L-R-
   INFORMATION FOR SEQUENCE ID NO 301
      (A) LENGTH:
      (B) TYPE: amino acid
      (D) TOPOLOGY: linear
         -H-E-R-E-E-E-L-R-K-R-L-R-
   INFORMATION FOR SEQUENCE ID NO 302
      (A) LENGTH:
      (B) TYPE: amino acid
      (D) TOPOLOGY: linear
         -T-E-E-E-L-R-R-R-L-R-
   INFORMATION FOR SEQUENCE ID NO 303
      (A) LENGTH:
      (B) TYPE: amino acid
      (D) TOPOLOGY: linear
         -N-E-E-E-L-R-R-R-L-R-
   INFORMATION FOR SEQUENCE ID NO 304
      (A) LENGTH:
      (B) TYPE: amino acid
      (D) TOPOLOGY: linear
         -E-R-A-D-E-E-G-L-Q-G-K-L-R-
   INFORMATION FOR SEQUENCE ID NO 305
      (A) LENGTH:
      (B) TYPE: amino acid
      (D) TOPOLOGY: linear
         -D-S-D-E-E-L-L-R-T-V-R-
   INFORMATION FOR SEQUENCE ID NO 306
      (A) LENGTH:
      (B) TYPE: amino acid
      (D) TOPOLOGY: linear
         -E-P-O-D-D-A-R-L-L-O-A-V-K-
   INFORMATION FOR SEQUENCE ID NO 307
      (A) LENGTH:
      (B) TYPE: amino acid
      (D) TOPOLOGY: linear
         -P-E-E-R-R-L-L-B-
   INFORMATION FOR SEQUENCE ID NO 308
      (A) LENGTH:
      (B) TYPE: amino acid
      (D) TOPOLOGY: linear
         -W-R-D-M-E-P-P-L-R-E-
   INFORMATION FOR SEQUENCE ID NO 309
      (A) LENGTH:
      (B) TYPE: amino acid
      (D) TOPOLOGY: linear
         -W-R-D-M-E-P-P-L-R-E-
   INFORMATION FOR SEQUENCE ID NO 310
      (A) LENGTH:
      (B) TYPE: amino acid
      (D) TOPOLOGY: linear
         -Q-K-S-M-E-P-P-L-R-E-
   INFORMATION FOR SEQUENCE ID NO 311
      (A) LENGTH
      (B) TYPE: amino acid
      (D) TOPOLOGY: linear
         -K-K-K-R-G-W-Y-K-W-L-R-K-L-
   INFORMATION FOR SEQUENCE ID NO 312
      (A) LENGTH:
      (B) TYPE: amino acid
      (D) TOPOLOGY: linear
         -K-R-R-K-G-W-F-Q-W-L-R-K-L-
**ANGIOTENSINOGEN**
   INFORMATION FOR SEQUENCE ID NO 313
      (A) LENGTH:
      (B) TYPE: amino acid
      (D) TOPOLOGY: linear
         -D-R-V-Y-I-H-P-F-H-L-L-Y-H-N-K-
   INFORMATION FOR SEQUENCE ID NO 314
      (A) LENGTH:
      (B) TYPE: amino acid
      (D) TOPOLOGY: linear
         -D-R-V-Y-I-H-P-F-H-L-L-Y-Y-S-K-
   INFORMATION FOR SEQUENCE ID NO 315
      (A) LENGTH:
      (B) TYPE: amino acid
      (D) TOPOLOGY: linear
         -D-R-V-Y-I-H-P-F-H-L-V-I-H-N-E-
   INFORMATION FOR SEQUENCE ID NO 316
      (A) LENGTH:
      (B) TYPE: amino acid
      (D) TOPOLOGY: linear
         -E-N-P-S-V-E-T-L-P-E-S-T-F-
   INFORMATION FOR SEQUENCE ID NO 317
      (A) LENGTH:
      (B) TYPE: amino acid
      (D) TOPOLOGY: linear
         -E-N-P-S-V-E-T-L-P-E-P-T-F-
   INFORMATION FOR SEQUENCE ID NO 318
      (A) LENGTH:
      (B) TYPE: amino acid
      (D) TOPOLOGY: linear
         -A-K-A-N-A-G-K-P-K-D-P-T-F-
   INFORMATION FOR SEQUENCE ID NO 319
      (A) LENGTH:
      (B) TYPE: amino acid
      (D) TOPOLOGY: linear
         -K-L-S-N-D-R-I-R-V-G-E-
   INFORMATION FOR SEQUENCE ID NO 320
      (A) LENGTH:
      (B) TYPE: amino acid
      (D) TOPOLOGY linear
         -K-M-G-D-T-N-P-R-V-G-E-
   INFORMATION FOR SEQUENCE ID NO 321
      (A) LENGTH:
      (B) TYPE: amino acid
      (D) TOPOLOGY: linear
         -N-I-G-D-T-N-P-R-V-G-E-
   INFORMATION FOR SEQUENCE ID NO 322
      (A) LENGTH:
      (8) TYPE: amino acid
      (D) TOPOLOGY: linear
         -E-L-E-A-D-E-R-E-P-T-E-
**PRORENIN**
   INFORMATION FOR SEQUENCE ID NO 323
      (A) LENGTH:
      (B) TYPE: amino acid
      (D) TOPOLOGY: linear
         -D-G-W-R-R-M-P-
   INFORMATION FOR SEQUENCE ID NO 324
      (A) LENGTH:
      (B) TYPE: amino acid
      (D) TOPOLOGY: linear
         -D-R-R-R-M-P-
   INFORMATION FOR SEQUENCE ID NO 325
      (A) LENGTH:
      (B) TYPE: amino acid
      (D) TOPOLOGY: linear
         -K-R-I-F-L-K-R-M-P-S-I-R-E-
   INFORMATION FOR SEQUENCE ID NO 326
      (A) LENGTH:
      (B) TYPE: amino acid
      (D) TOPOLOGY: linear
         -G-R-I-L-L-K-K-M-P-S-V-R-E-
   INFORMATION FOR SEQUENCE ID NO 327
      (A) LENGTH:
      (B) TYPE: amino acid
      (D) TOPOLOGY: linear
         -K-R-M-P-S-I-R-E-S-L-K-E-R-G-
   INFORMATION FOR SEQUENCE ID NO 328
      (A) LENGTH:
      (B) TYPE: amino acid
      (D) TOPOLOGY: linear
         -K-K-M-P-S-V-R-E-I-L-E-E-R-G-
   INFORMATION FOR SEQUENCE ID NO 329
      (A) LENGTH:
      (B) TYPE: amino acid
      (D) TOPOLOGY: linear
         -R-E-I-L-E-E-R-G-
   INFORMATION FOR SEQUENCE ID NO 330
      (A) LENGTH:
      (B) TYPE: ammo acid
      (D) TOPOLOGY: linear
         -R-L-G-P-E-W-S-Q-P-M-K-R-
   INFORMATION FOR SEQUENCE ID NO 331
      (A) LENGTH:
      (B) TYPE: amino acid
      (D) TOPOLOGY: linear
         -R-I-S-A-E-W-G-E-F-I-K-K-
   INFORMATION FOR SEQUENCE ID NO 332
      (A) LENGTH:
      (B) TYPE: amino acid
      (D) TOPOLOGY: linear
         -R-I-S-A-E-W-G-E-F-I-K-K-
   INFORMATION FOR SEQUENCE ID NO 333
      (A) LENGTH:
      (B) TYPE: amino acid
      (D) TOPOLOGY: linear
         -R-L-S-A-E-W-G-V-F-T-K-R-
   INFORMATION FOR SEQUENCE ID NO 334
      (A) LENGTH:
      (B) TYPE: amino acid
      (D) TOPOLOGY: linear
         -H-K-L-F-D-A-S-D-S-S-S-Y-K-H-
   INFORMATION FOR SEQUENCE ID NO 335
      (A) LENGTH:
      (B) TYPE: amino acid
      (D) TOPOLOGY: linear
         -H-N-L-Y-D-S-S-E-S-S-S-Y-M-E-
   INFORMATION FOR SEQUENCE ID NO 336
      (A) LENGTH:
      (B) TYPE: amino acid
      (D) TOPOLOGY: linear
         -H-S-L-Y-E-S-S-D-S-S-S-Y-M-E-
   INFORMATION FOR SEQUENCE ID NO 337
      (A) LENGTH:
      (B) TYPE: amino acid
      (D) TOPOLOGY: linear
         -K-E-D-V-F-S-F-S-F-Y-Y-N-R-D-S-E-N-
   INFORMATION FOR SEQUENCE ID NO 338
      (A) LENGTH:
      (B) TYPE: amino acid
      (D) TOPOLOGY: linear
         -K-E-E-V-F-S-V-Y-Y-N-R-G-S-H-
   INFORMATION FOR SEQUENCE ID NO 339
      (A) LENGTH:
      (B) TYPE: amino acid
      (D) TOPOLOGY: linear
         -D-P-Q-H-Y-E-G-N-F-H-
   INFORMATION FOR SEQUENCE ID NO 340
      (A) LENGTH:
      (B) TYPE: amino acid
      (D) TOPOLOGY: linear
         -E-K-L-M-E-A-L-G-A-K-K-R-L-F-D-
   INFORMATION FOR SEQUENCE ID NO 341
      (A) LENGTH:
      (B) TYPE: amino acid
      (D) TOPOLOGY: linear
         -Q-L-I-M-Q-A-L-G-V-K-E-K-R-A-N-
   INFORMATION FOR SEQUENCE ID NO 342
      (A) LENGTH:
      (B) TYPE: amino acid
      (D) TOPOLOGY: linear
         -K-L-I-M-Q-A-L-G-A-K-E-K-R-I-E-
   INFORMATION FOR SEQUENCE ID NO 343
      (A) LENGTH:
      (B) TYPE: amino acid
      (D) TOPOLOGY: linear
         -D-Y-V-F-Q-E-S-Y-S-S-K-K-
   INFORMATION FOR SEQUENCE ID NO 344
      (A) LENGTH:
      (B) TYPE: amino acid
      (D) TOPOLOGY: linear
         -D-Y-V-Q-K-N-P-F-R-N-D-D-
   INFORMATION FOR SEQUENCE ID NO 345
      (A) LENGTH:
      (B) TYPE: amino acid
      (D) TOPOLOGY: linear
         -D-Y-V-Q-Y-P-N-R-R-D-K-
   INFORMATION FOR SEQUENCE ID NO 346
      (A) LENGTH:
      (B) TYPE: amino acid
      (D) TOPOLOGY: linear
         -R-K-F-Y-T-E-D-R-R-N-N-R-
   INFORMATION FOR SEQUENCE ID NO 347
      (A) LENGTH:
      (B) TYPE: amino acid
      (D) TOPOLOGY: linear
         -R-K-F-Y-T-E-F-D-R-H-N-N-R-
   INFORMATION FOR SEQUENCE ID NO 348
      (A) LENGTH:
      (B) TYPE: amino acid
      (D) TOPOLOGY: linear
         -R-K-F-Y-T-E-F-D-R-H-N-N-R-
**CORTICOTROPIN RELEASING FACTOR BINDING PROTEIN**
   INFORMATION FOR SEQUENCE ID NO 349
      (A) LENGTH:
      (B) TYPE: amino acid
      (D) TOPOLOGY: linear
         -R-Y-L-E-L-R-E-A-A-D-Y-D-
   INFORMATION FOR SEQUENCE ID NO 350
      (A) LENGTH:
      (B) TYPE: amino acid
      (D) TOPOLOGY: linear
         -R-Y-L-E-V-Q-E-A-A-V-Y-D-
   INFORMATION FOR SEQUENCE ID NO 351
      (A) LENGTH:
      (B) TYPE: amino acid
      (D) TOPOLOGY: linear
         -K-R-D-V-A-G-E-Q-P-Y-R-R-
   INFORMATION FOR SEQUENCE ID NO 352
      (A) LENGTH:
      (B) TYPE: amino acid
      (D) TOPOLOGY: linear
         -K-R-N-L-A-E-E-Q-P-Y-R-R-
   INFORMATION FOR SEQUENCE ID NO 353
      (A) LENGTH:
      (B) TYPE: amino acid
      (D) TOPOLOGY: linear
         -D-H-P-L-P-S-A-E-R-
   INFORMATION FOR SEQUENCE ID NO 354
      (A) LENGTH:
      (B) TYPE: amino acid
      (D) TOPOLOGY: linear
         -D-H-P-L-P-T-R-E-R-
   INFORMATION FOR SEQUENCE ID NO 355
      (A) LENGTH:
      (B) TYPE: amino acid
      (D) TOPOLOGY: linear
         -R-R-S-I-R-S-S-Q-
   INFORMATION FOR SEQUENCE ID NO 356
      (A) LENGTH:
      (B) TYPE: amino acid
      (D) TOPOLOGY: linear
         -R-R-S-V-T-S-S-Q-
   INFORMATION FOR SEQUENCE ID NO 367
      (A) LENGTH:
      (B) TYPE: amino acid
      (D) TOPOLOGY: linear
         -D-P-S-K-M-T-P-L-A-D-
   INFORMATION FOR SEQUENCE ID NO 358
      (A) LENGTH:
      (B) TYPE: amino acid
      (D) TOPOLOGY: linear
         -D-T-S-K-M-M-L-L-V-D-
   INFORMATION FOR SEQUENCE ID NO 359
      (A) LENGTH:
      (B) TYPE: amino acid
      (D) TOPOLOGY: linear
         -E-P-Y-E-L-E-N-P-N-G-
   INFORMATION FOR SEQUENCE ID NO 360
      (A) LENGTH:
      (B) TYPE: amino acid
      (D) TOPOLOGY: linear
         -E-P-L-E-L-E-T-S-T-R-

## Claims

1. Use of a method for identifying synthetic signal oligopeptides as direct competitive inhibitors to specifically prevent or reduce the metabolic action or interaction of a selected protein by blocking its specific signal sequences said method comprising identifying a protein responsible for causing human disease, identifying one or more signal oligopeptide sequences within the structure of the disease causing protein, identifying one or more signal oligopeptides being the amino acid sequence of maximum hydrophilicity and/or surface probability and/or electrical charge of the protein, synthesizing one or more oligopeptides having sequences corresponding to the amino acid sequences of the signal oligopeptides of maximum hydrophilicity and/or surface probability and/or electrical charge of the protein.

2. The use of a method according to claim 1 wherein the synthetic oligopeptides are analogous to the specific oligopeptides no. 1 - 360 of the attached sequence listing.

## Patentansprüche

1. Verwendung eines Verfahrens zur Identifizierung synthetischer Signaloligopeptide als direkte kompetitive Inhibitoren um spezifisch der metabolischen Funktion oder Interaktion eines ausgewählten Proteins durch Blockierung seiner spezifischen Signalsequenzen vorzubeugen oder diese zu reduzieren, wobei die Methode das Identifizieren eines Proteins, das verantwortlich für die Verursachung einer Erkrankung im Menschen ist, das Identifizieren einer oder mehrerer Signaloligopeptidsequenzen innerhalb der Struktur dieses die Erkrankung verursachenden Proteins, das Identifizieren eines oder mehrerer Signaloligopeptide, die die Aminosäuresequenzen mit maximaler Hydrophilie und/oder Oberflächenwahrscheinlichkeit und/oder elektrischer Ladung des Proteins sind, das Synthetisieren eines oder mehrerer Oligopeptide, die Sequenzen aufweisen, die zu den Aminosäuresequenzen der Signaloligopeptide mit maximaler Hydrophilie und/oder Oberflächenwahrscheinlichkeit und/oder elektrischer Ladung des Proteins korrespondieren umfasst.

2. Verwendung nach Anspruch 1, wobei die synthetischen Oligopeptide analog zu den spezifischen Oligopeptiden Nr. 1 bis 360 des beigefügten Sequenzprotokolls sind.

## Revendications

1. Utilisation d'une méthode pour l'identification d'oligopeptides de signal synthétiques comme inhibiteurs directs performants pour empêcher ou réduire spécifiquement l'action ou l'interaction métabolique d'une protéine sélectionnée en bloquant ses séquences de signal spécifiques, ladite méthode comprend :
- identification d'une protéine responsable pour la cause de maladie humaine,
- identification d'une ou plusieurs séquences oligopeptides de signal au sein de la structure de protéine provoquant la maladie,
- identification d'une ou plusieurs oligopeptides de signal qui sont les séquences d'acide aminée avec une hydrophobicité maximum et/ou une probabilité de surface et/ou charge électrique de la protéine,
- synthèse d'une ou plusieurs oligopeptides ayant des séquences correspondantes aux séquences d'acide aminée de l'oligopeptide de signal d'une hydrophobicité maximum et/ou probabilité de surface et/ou charge électrique de la protéine.

2. L'utilisation d'une méthode selon la revendication 1 sachant que les oligopeptides synthétiques sont analogues aux oligopeptides spécifiques n° 1 à 360 de la liste de séquences jointe.
